# EUROPEAN PATENT APPLICATION

(11) **EP 4 327 768 A1**
(43) Date of publication of application: **28.02.2024**
(21) Application number: 23192582.7
(22) Date of filing: 22.08.2023
(51) Int. Cl.: A61B 18/14, A61B 5/00, A61B 5/287

(54) **PLANAR MULTI-ELECTRODE CATHETERS**

(30) Priority: 23.08.2022 US 202263400184 P; 02.09.2022 US 202263403589 P; 14.09.2022 US 202263406673 P; 07.08.2023 US 202318230934
(71) Applicant: BIOSENSE WEBSTER (ISRAEL) LTD., 2066717 Yokneam (IL)
(72) Inventor: EBRAHIMI, Babak, Irvine, 92618 (US); BASU, Shubhayu, Irvine, 92618 (US); BAR-TAL, Meir, 2066717 Yokneam (IL); RODRIGUEZ SOTO, Juan, Irvine, 92618 (US); VAN NIEKERK, Pieter Emmelius, Irvine, 92618 (US); HIGHSMITH, Debby, Irvine, 92618 (US); ABBAS, Mohammad, Irvine, 92618 (US); BERGER, Abraham, 2066717 Yokneam (IL)
(74) Representative: Carpmaels & Ransford LLP

(57) **Abstract**

Catheters are presented herein having planar end effectors of various configurations, generally providing a two-sided and multi-layered platform for electrodes and sensors. In some examples, the end effector has a support frame (e.g. nitinol) between a pair of flexible circuits. The end effector can also include a polymer (e.g. silicone, LCP, etc.) between the flexible circuits and encapsulating the support frame. This platform facilitates positioning of electrodes on either side (including both sides) of the end effector in a variety of spacings and facilitates ultra-tight electrode spacing and/or a large area electrode. Sensors (ultrasound transducers, navigation coils, etc.) can be layered within the end effector between outer surfaces of the flexible circuits in a variety of configurations.

## Description

### CROSS REFERENCE TO RELATED APPLICATIONS

This application claims the benefit of priority to prior filed U.S. Provisional Patent Application Nos. 63/400,184 filed August 23, 2022, 63/403,589 filed September 2, 2022, and 63/406,673 filed September 14, 2022, each of which are hereby incorporated by reference as if set forth in full herein.

### FIELD

This application relates generally to multi-electrode catheters for diagnostic and therapeutic purposes and particularly to planar multi-electrode catheters.

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals can be located in tissue of an atria or a ventricle. Unwanted signals are conducted elsewhere through heart tissue where they can initiate or continue arrhythmia.

Procedures for treating arrhythmia include surgically disrupting the origin of the signals causing the arrhythmia, as well as disrupting the conducting pathway for such signals. More recently, it has been found that by mapping the electrical properties of the endocardium and the heart volume, and selectively ablating cardiac tissue by application of energy, it is possible to cease or modify the propagation of unwanted electrical signals from one portion of the heart to another. The ablation process destroys the unwanted electrical pathways by formation of non-conducting regions of tissue.

In this two-step procedure, which includes mapping followed by ablation, electrical activity at points in the heart is typically sensed and measured by advancing a catheter containing one or more electrical sensors into the heart and acquiring data at multiple points. These data are then utilized to select the target areas at which ablation is to be performed.

For greater mapping resolution, it is desirable for a mapping catheter to provide high-density signal maps through the use of several electrodes sensing electrical activity of tissue in an area on the order of a square centimeter.

### SUMMARY

Catheters are presented herein having planar end effectors of various configurations, generally providing a two-sided and multi-layered platform for electrodes and sensors. In some examples, the end effector has a support frame (e.g. nitinol) between a pair of flexible circuits. The end effector can also include a polymer (e.g. silicone, LCP, etc.) between the flexible circuits and encapsulating the support frame. This platform facilitates positioning of electrodes on either side (including both sides) of the end effector in a variety of spacings and facilitates ultra-tight electrode spacing. Sensors (ultrasound transducers, navigation coils, etc.) can be layered within the end effector between outer surfaces of the flexible circuits in a variety of configurations.

An example end effector of a catheter can include a frame, a first membrane, a plurality of first electrodes, a second membrane, and a plurality of second electrodes. The frame can include one or more struts extending along a longitudinal axis. The first membrane can extend between the one or more struts on a first side of the end effector. The first membrane can define a generally planar first surface exposed to ambient environment. The plurality of first electrodes can be affixed to the first membrane at predetermined locations on the first surface. The second membrane can extend between the one or more struts such that at least a portion of the frame is between the first membrane and the second membrane. The second membrane can define a generally planar second surface exposed to the ambient environment. The plurality of second electrodes can be affixed to the second exposed surface so that each second electrode is disposed substantially opposite each first electrode and spaced apart from each other.

The frame can be symmetric about the longitudinal axis.

The one or more struts can include a first strut and a second strut. The first strut can be positioned between the first membrane and the second membrane, and the second strut can be positioned between the first membrane and the second membrane.

The first and second struts can be symmetric about the longitudinal axis.

The one or more struts can include a proximal strut and a pair of struts extending distally from the proximal strut. The pair of struts can each terminate at a respective distal strut end.

The proximal strut can have a width greater than a width of each strut of the pair of struts.

The one or more struts can include a proximal strut and between two and twelve struts extending distally from the proximal strut.

The one or more struts can include one or more looped struts positioned approximate a perimeter of the end effector. The one or more looped struts can include a connected looped strut and/or a disconnected looped strut. The end effector can include electrical conductors in electrical contact with the looped strut and extending proximally from the frame. The looped strut can be symmetric about the longitudinal axis. The looped strut can have a first proximal end, a second proximal end proximal, and a continuous, non-branching path extending from the first proximal end to the second proximal end. The looped strut can have a pair of parallel segments extending parallel to the longitudinal axis and a connector segment extending across the longitudinal axis and joining segments of the pair of parallel segments.

At least a portion of the one or more struts can have a flexibility gradient along a portion of a length of the respective strut.

At least a portion of the one or more struts can have a graduated cutout pattern along a portion of a length of the respective strut.

At least one of the first membrane and the second membrane can include a flex circuit.

The first membrane can include electrically conductive traces in electrical contact with the plurality of first electrodes. The second membrane can include electrically conductive traces in electrical contact with the plurality of second electrodes.

The first membrane and the second membrane can each include a respective proximal extension extending proximally from a proximal end of the frame. The proximal extensions can have a length of between about 30 centimeters (cm) and about 150 cm. The proximal extensions can have a length of between about 60 centimeters (cm) and about 130 cm. The proximal extensions can include a plurality of conductive traces in electrical contact with the plurality of first electrodes on the first surface of the end effector and the plurality of second electrodes on the second surface of the end effector.

At least a portion of the plurality of first electrodes can be positioned to contact tissue.

At least a portion of the plurality of first electrodes can be positioned between the one or more struts.

The plurality of first electrodes can be positioned to have a gap between a pair of coplanar electrodes measuring about 5 micrometers to about 650 micrometers, or more preferably, a gap about 150 micrometers.

The plurality of first electrodes can include a plurality of closely spaced coplanar electrode pairs. The closely spaced coplanar electrode pairs can be spaced with a pitch of between about of about 0.5 millimeters to about 6 millimeters or more preferably a pitch of between about 2.5 millimeters and about 5 millimeters between pairs and a gap of about 150 micrometers between electrodes of a pair.

The first membrane can include a first longitudinally extending section having a first width. A first electrode that is affixed to the first membrane can be disposed centrally in relation to the first width. The first membrane can include a second longitudinally extending section having a second width that is greater than the first width. A second electrode that is affixed to the first membrane can be disposed centrally in relation to the second width.

The plurality of second electrodes can include a reference electrode.

The plurality of second electrodes can include a plurality of reference electrodes each configured to provide a reference signal to a respective electrode of the plurality of first electrodes.

The plurality of first electrodes can be distributed in a high electrode density zone and low electrode density zone. The high electrode density zone can be disposed distal of the low electrode density zone. The high electrode density zone can be of approximately equal area as the low electrode density zone.

The end effector can further include a reference electrode positioned on a portion of the end effector that is configured to be positioned approximate a distal end of a catheter shaft.

The end effector can further include a polymer encapsulating the frame.

The end effector can further include a polymer positioned between the first membrane and the second membrane.

The end effector can further include a navigation sensor positioned such that at least a portion of the navigation sensor is between the one or more struts. The navigation sensor can have an area of about 16 millimeters squared. The navigation sensor can be configured as a single axis sensor or a double axis sensor. The navigation sensor can be planar or non-planar.

The end effector can further include one or more piezoelectric transducers. The one or more piezoelectric transducers can be disposed under a respective electrode of the plurality of electrodes affixed to the first membrane. The one or more piezoelectric transducers can be positioned within an opening in a strut of the one or more struts. The one or more piezoelectric transducers can be positioned between the first membrane, between the second membrane, and between the one or more struts. The one or more piezoelectric transducers can include lead zirconate titanate (PZT). The one or more piezoelectric transducers can be configured as an ultrasound transducer.

Each of the first membrane and second membrane can include a continuous single member.

Each of the first membrane and second membrane can include a cut-out disposed on at least one side of the longitudinal axis.

The end effector can include a pair of longitudinal openings separating a central portion of the end effector from a left portion of the end effector and a right portion of the end effector. The frame can be positioned in the left portion and the right portion. The frame can be absent in the central portion. The end effector can be configured for delivery through a catheter having an inner diameter, and the central portion can have a width approximately equal to the inner diameter of the catheter. The left portion and the right portion can be configured to fold toward the central portion for delivery of the end effector through a catheter. A majority of the plurality of first electrodes can be disposed on the central portion of the end effector. A first portion of the plurality of first electrodes can be disposed on the right portion of the end effector. A second portion of the plurality of first electrodes can be disposed on the left portion of the end effector. The central portion can include a plurality of openings each being longitudinally shorter in length than each of the pair of longitudinal openings. The frame can include a first distal end positioned in the left portion. The frame can include a second distal end positioned in the right portion. The first membrane and/or the second membrane can extend over the first distal end of the frame, across a distal end of the end effector, and over the second distal end of the frame. The first membrane and/or the second membrane can extend in a single arc from the first distal end of the frame, across the distal end of the end effector, and to the second distal end of the frame. Additionally, or alternatively, the first membrane and/or the second membrane can extend in a first arc from the first distal end of the frame to the central portion of the end effector, a second arc across a distal end of the central portion, and a third arc from the central portion to the second distal end of the frame.

The end effector can have a curvature defining an arcuate path around to the longitudinal axis when the end effector is in free space.

The end effector can have a planar shape when pressed to a planar surface.

An example catheter control system can include a processor and non-transitory computer readable medium in communication with the processor and comprising instructions thereon that when executed by the processor causes the system to: receive a mapping electrical signal from a mapping electrode such that the mapping electrode faces tissue in a first direction and is disposed on a first side of an end effector, and receive a reference electrical signal from a reference electrode such that the reference electrode faces away from the tissue in a second direction and is disposed on a second side of the end effector opposite the first side.

The instructions can further be executed to cause the system to receive an ultrasound electrical signal from an acoustic transducer disposed in a plane that is between the mapping electrode and the reference electrode.

The instructions can further be executed to utilize a plurality of tomography electrodes disposed on the first side of the end effector to perform impedance tomography of the tissue. The instructions can be executed to apply an alternating current to a first plurality of tomography electrodes disposed on the first side of the end effector, receive equi-potentials of a second plurality of tomography electrodes paired with the first plurality of tomography electrodes and disposed on the first side of the end effector, generate a tomographic image of the tissue based on the equi-potentials, and display the tomographic image on a display.

The instructions can further be executed to receive a bipolar mapping electrical signal from a first pair of bipolar electrodes facing the first direction on the first surface, and receive the reference electrical signal from a second pair of reference electrodes disposed opposite the first pair of bipolar electrodes such the second pair of reference electrodes face the second direction opposite the first direction on the second side of the end effector.

The instructions can further be executed to receive a navigation signal from a single axis sensor coil disposed in a plane that is between the mapping electrode and the reference electrode.

The instructions can further be executed to determine a positioned of the end effector based at least in part on an impedance between an electrode of the end effector and a body patch electrode.

The instructions can further be executed to receive a navigation signal from a single axis sensor coil disposed at a distal end of a shaft of the catheter and disposed in an off-angle plane that is at an angle to an end effector plane that is between the mapping electrode and the reference electrode.

Another example end effector configured for a catheter can include a frame structure having first and second segments (S1, S2) extending alongside each other in a direction along a longitudinal axis. Each segment can have a first outer surface and a second outer surface facing away in a generally opposite direction to the first outer surface. The first outer surface can include at least one first pair of closely-spaced electrodes in which each first pair of closely-spaced electrodes includes two electrodes spaced apart over any gap length of approximately 0.1 to approximately 0.4 mm as measured from one electrode edge of one electrode to a nearest electrode edge of the other closely-spaced electrode. The second outer surface can include at least a second pair of closely-spaced electrodes in which each second pair of closely-spaced electrodes includes two electrodes spaced apart over any gap length of approximately 0.1 mm to approximately 0.4 mm from one electrode edge of one electrode to a nearest electrode edge of the other closely-spaced electrode.

The first outer surface can have a surface substantially parallel to the second outer surface.

The two segments can be connected to each other at one end to form a conductive loop. The conductive loop can have electrical terminations at a proximal portion so that the conductive loop includes an electromagnetic sensor.

The at least one first pair of closely-spaced electrodes on the first surface can include two pairs of closely spaced electrodes in which one pair of closely-spaced electrodes is separated to the other pair of closely-spaced electrodes by a gap length (Ls) measured from the closest edges of respective electrodes of any value from approximately 0.5mm to 1.5 mm.

The at least one second pair of closely-spaced electrodes on the second surface can include two pairs of closely spaced electrodes in which one pair of closely-spaced electrodes is separated to the other pair of closely-spaced electrodes by a gap length (Ls) measured from the closest edges of respective electrodes of any value from approximately 0.5mm to 2 mm.

Each electrode can have a surface area of any value from approximately 0.04 mm-squared to 1 mm-squared in surface area exposed to ambient environment.

Each electrode can have a rectangular area exposed to ambient environment having a length along the longitudinal axis of any value from approximately 0.1 mm to approximately 1 mm and a width of any value from approximately 0.1 mm to approximately 1 mm.

Each electrode can have a square area exposed to ambient environment having a side of any value from approximately 0.1 mm to approximately 1 mm.

Each electrode can have a non-linear surface area.

Each electrode on the first outer surface can include an electrode surface exposed to the ambient environment parallel to a counterpart electrode surface of a counterpart electrode disposed on the second outer surface.

Each segment can have a linear cross-sectional area orthogonal to the longitudinal axis.

Each segment can have a non-linear cross-sectional area orthogonal to the longitudinal axis.

Each electrode can have a linear cross-sectional area orthogonal to the longitudinal axis. The area can measure from about 0.00005mm-squared to about 0.005 mm-squared.

Each electrode can have a non-linear cross-sectional area orthogonal to the longitudinal axis.

The end effector can further include another frame structure having third and fourth segments extending alongside each other and the first and second segments so that the third segment is disposed between the first and second segments and the second segment is disposed between the third and fourth segments.

An electrode on one segment is spaced apart from a center of the electrode to a center of another electrode on adjacent segment over a first transverse gap having any value from approximately 0.5 mm to approximately 4 mm.

An electrode on one segment can be spaced apart from the electrode edge to a nearest electrode edge of another electrode on adjacent segment over a second transverse gap having any value from approximately 0.3mm to approximately 3.8 mm.

One segment can be spaced apart from another segment along the transverse axis over a third transverse gap of less than the second transverse gap.

All the segments can be parallel to each other.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
Figures 1A, 1B, 1C, 1D, 1E, 1F, and 1G are illustrations of various views and components of a first example end effector.
Figures 2A, 2B, 2C, 2D and 2E are illustrations of various view and components of a second example end effector.
Figure 3 is an illustration of planar view of a third example end effector.
Figures 4A, 4B, and 4C are illustrations of example frame configurations.
Figure 5 is a flow diagram of a method for constructing a catheter.
Figure 6 is an illustration of a medical procedure utilizing a catheter and a catheter control system.
Figures 7A, 7B, 7C, 7D, and 7E are illustrations of an example catheter including another example end effector.
Figure 7F is an illustration of a plural loop version of the single loop end effector illustrated in Figures 7A through 7E.
Figure 7G is a close-up illustration of an outer surface of the end effector including bipole electrode pairs as indicated in Figure 7F.
Figures 7H and 7I are cross-sectional illustrations of the end effector as indicated in Figure 7F.
Figure 7J is a perspective view illustration of a variation of the end effector illustrated in Figure 7F.
Figure 7K is an illustration of electrode spacing on an end effector as illustrated in Figure 7J.
Figure 7L is an illustration of an ECG recording of cardiac tissue adjacent an image of the catheter position in a heart.
Figure 8 is an illustration of another example end effector.
Figure 9 is an illustration of another example end effector.
Figure 10A is an illustration of another example end effector having a large area electrode.
Figures 10B and 10C is an illustrations of example large area electrode geometries of the example end effector illustrated in Figure 10A.
Figure 10D is an illustration of an example simulation of pulse field ablation through 10C.
Figure 10E is an illustration of a variation of the example end effector of Figures 10A through 10C including smaller, independent electrodes.
Figure 10F is a variation of Fig. 10E in which the electrical traces are thinner with larger radii of curvatures and different electrodes disposed thereon;
Figure 11 is an illustration of another example end effector including multiple membrane structures.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%.

In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator whereas "distal" indicates a location further away to the operator or physician.

As discussed herein, the term "irreversible electroporation (IRE)" or "pulse field ablation (PFA)", and "pulsed electric field (PEF) ablation" are used interchangeably herein to refer to application of electrical signals to cardiac tissue to reduce or prevent the generation of erratic cardiac signals in the cells by utilizing short duration electrical pulses intended to provide electric fields across cellular membranes of target tissue to result in irreversible electroporation of the target tissue cellular membranes. IRE signals can include biphasic pulses including a positive-voltage pulse followed by a negative-voltage pulse. IRE signals can include monophasic pulses including only one voltage pulse polarity. IRE signals can be applied in a monopolar configuration in which the IRE signals are applied between an electrode applied to the target tissue, and one or more remote electrodes (e.g. body patch electrode(s) attached to a patient's skin and having a larger total surface area than the electrode at target tissue). IRE signals can be applied in a bipolar configuration in with IRE signals are applied between two electrodes applied to the target tissue, or near the target tissue.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the pertinent art in view of the teachings herein. Such modifications and variations are intended to be included within the scope of the claims.

Example end effectors are illustrated and disclosed herein which are generally planar and include multiple electrodes that can be configured for mapping and/or ablation. The end effectors can be joined to a shaft with additional catheter components to form a mapping and/or ablation catheter through processes disclosed herein and processes similar to those known by a person skilled in the pertinent art. The example end effectors illustrated herein include variations and features that are combinable to form additional end effector designs as understood by a person skilled in the pertinent art.

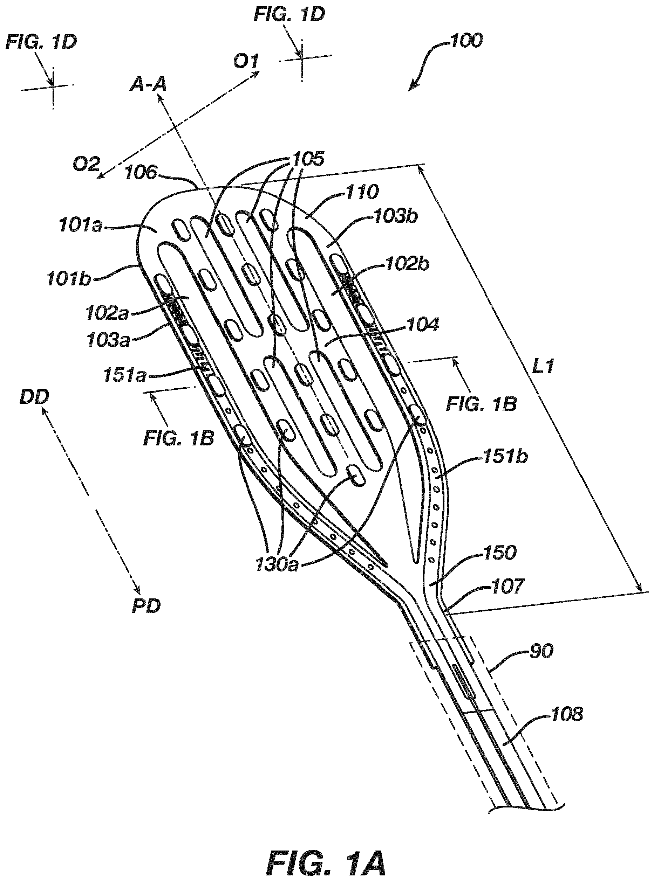

Figures 1A through 1G are illustrations of various views and components of a first example end effector 100.

Figure 1A is an isometric view of the first example end effector 100. The end effector 100 extends along a longitudinal axis A-A and is substantially planar, with a first surface 101a and an opposite second surface 101b. The first surface 101a and the second surface 101b can each be exposed to an ambient environment so that either surface 101a, 101b can be pressed against tissue. The first surface faces a first direction O1 that is orthogonal to the longitudinal axis A-A and orthogonal to a plane defined by the end effector 100. The second surface 101b faces a second direction O2 that is orthogonal to the longitudinal axis A-A and orthogonal to a plane defined by the end effector 100.

The end effector 100 is further oriented as having a distal end 106, a proximal portion 107 that can be positioned at a distal end of a catheter shaft, and a proximal extension 108 that can extend through at least a portion of a catheter shaft 90 (indicated in dash outline). The end effector 100 includes an exposed portion having a length L1. The exposed portion extends from a distal end 106 of the end effector 100 to a distal end of the catheter shaft 90 at the proximal portion 107 of the end effector 100.

The end effector 100 includes a frame 150 that can include struts 151a, 151b that extend along the longitudinal axis A-A, such that at least a portion of each strut 151a, 151b is parallel to the longitudinal axis A-A. The frame 150 is symmetric about the longitudinal axis A-A. The frame 150 supports a first membrane 110 that extends between the struts 151a, 151b of the frame 150. The membrane 110 can generally define the first planar surface 101a of the end effector 100.

The membrane 110 supports the surface electrodes 130a and the end effector 100 need not include frame struts under the surface electrodes 130a. The electrodes 130a can be affixed at predetermined locations on the first surface 101a of the end effector 100 and spaced apart from each other.

The end effector 100 includes a left longitudinal opening 102a and a right longitudinal opening 102b that extend along a longitudinal axis A-A for a majority of the length L1 of the exposed end of the end effector 100. The left longitudinal opening 102a separates a left portion 103a of the end effector 100 from a central portion 104 of the end effector 100. The right longitudinal opening 102b separates a right portion 103b of end effector 100 from the central portion 104. The central portion 104 further includes longitudinal openings 105 that are shorter in length than the left longitudinal opening 102a and the right longitudinal opening 102b.

A majority of the surface electrodes 130a on the first surface 101a are positioned on the central portion 104 of the end effector. Surface electrodes 130a are also positioned on both the right portion 103b and the left portion 103a of the end effector 100.

The frame 150 extends into the left portion 103a and the right portion 103b. The surface electrodes 130a in the left portion 103a and the right portion 103b are positioned primarily over a left frame strut 151a and a right frame strut 151b. The electrodes 130a in the central portion 104 are not supported directly by the frame 150. The frame 150 does not extend into the central portion 104.

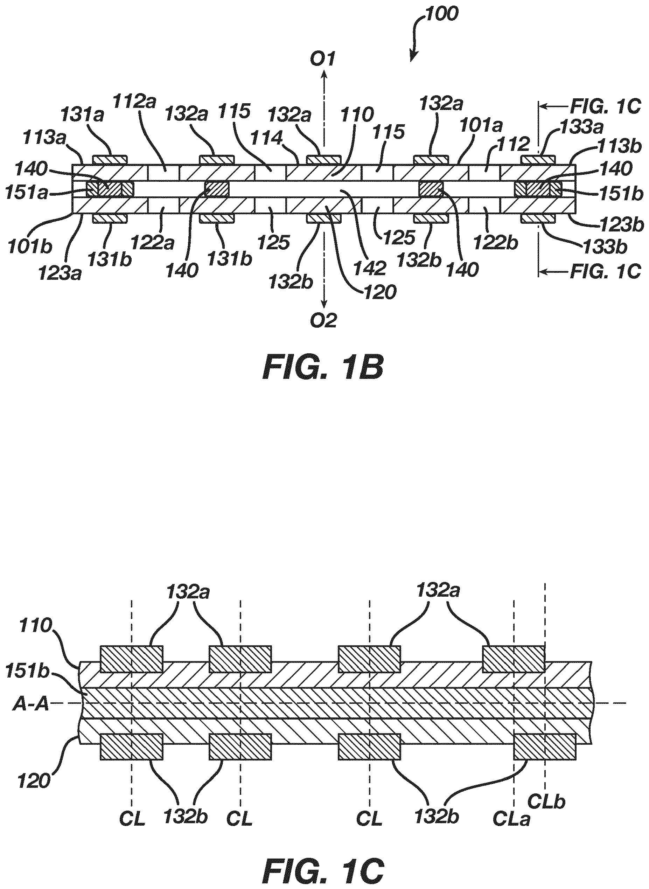

Figure 1B is a cross-sectional illustration of the end effector 100 as indicated in Figure 1A. The cross-section illustrates the first membrane 110 over struts 151a, 151b of the frame 150, longitudinal openings 102a, 102b, and surface electrodes 130a on the first surface 101a as illustrated in Figure 1A. The cross-sectional illustration also illustrates a second membrane 120, a polymer layer 142, and piezoelectric transducers 140.

Longitudinal openings 102a, 102b illustrated in Figure 1A include longitudinal openings 112a, 112b in the first membrane 110 and longitudinal openings 122a, 122b in the second membrane 120 as indicated in Figure 1B. The left portion 103a of the end effector 100 as indicated in Figure 1A includes left portions 113a, 123a of the first membrane 110 and the second membrane 120 as indicated in Figure 1B. The right portion 103b of the end effector 100 as indicated in Figure 1A includes right portions 113b, 123b of the first membrane 110 and the second membrane 120 as indicated in Figure 1B. The central portion 104 of the end effector 100 as indicated in Figure 1A includes central portions 114, 124 of the first membrane 110 and the second membrane 120 as indicated in Figure 1B. The central longitudinal openings 105 in the central portion 104 of the end effector 100 as indicated in Figure 1A includes central longitudinal openings 115, 125 in the first membrane 110 and the second membrane 120.

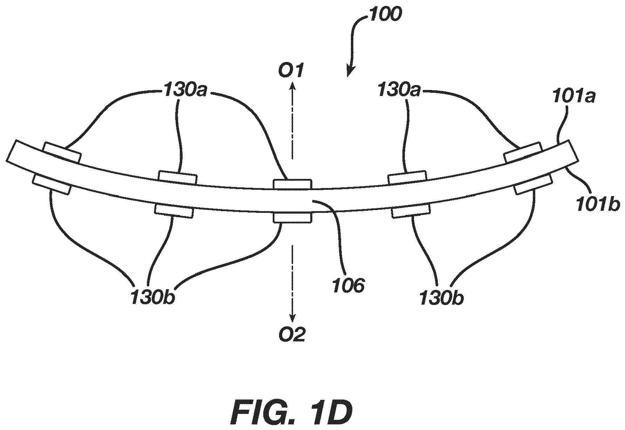

Electrodes 130a on the first surface 101a as indicated in Figure 1A are indicated as an electrode 131a on a left portion 113a of the first membrane, electrodes 132a on the central portion 114 of the first membrane 110, and electrodes 133a on the right portion 113b of the first membrane 110. The end effector 100 also includes electrodes 130b (see Figure 1D) on the second membrane 120 on the second surface 101b of the end effector 100 that include electrodes 131b on the left portion 123a of the second membrane 120, electrodes 132b on the central portion 124 of the second membrane 120, and electrodes 133b on the right portion 123b of the second membrane 120.

The second membrane 120 can be configured similarly to the first membrane 110. The second membrane 120 can be supported by the frame 150 and extend between struts 151a, 151b of the frame 150. At least a portion of the frame 150, including struts 151a, 151b can be positioned between the first membrane 110 and the second membrane 120. The second membrane 120 can define the second planar surface 101b of the end effector 100. The electrodes 130b on the second membrane 120 can be affixed at predetermined locations on the second surface 101b of the end effector 100 and spaced apart from each other. Some or all of the surface electrodes 130b on the second membrane 120 can be positioned opposite a respective surface electrode 130a on the first membrane 110. The electrode pattern on the second surface 101b can be, but need not be, identical to the electrode pattern on the first surface 101a.

The end effector 100 can include a structural member 142 positioned between the first membrane 110 and the second membrane 120, and positioned around the struts 151a, 151b of the frame 150. As illustrated, the membranes 110, 120 are indirect contact with the struts 151a, 151b; however, the membranes 110, 120 may be separated from the struts 151a, 151b in the orthogonal directions O1, O2 and the structural member 142 can be positioned between the struts 151a, 151b and the membranes 110, 120 to encapsulate the struts 151a, 151b.

The membranes 110, 120 can each respectively include a flex circuit. The membranes 110, 120 can respectively include electrically conductive traces in electrical contact with the electrodes 131a, 131b, 132a, 132b, 133a, 133b.

The end effector 100 can include piezoelectric transducers 140 positioned in a plane that is between the electrodes 130a on the first surface 101a and the electrodes 130b on the second surface 101b. A piezoelectric transducer 140 can be positioned under a respective electrode 130a, 130b or between opposite electrodes 130a, 130b; however a piezoelectric transducer 140 need not be positioned under an electrode 130a, 130b. A piezoelectric transducer 140 can be positioned within an opening of a strut 151a, 151b as illustrated in the left portion 103a and the right portion 103b of the end effector 100. Additionally, or alternatively, a piezoelectric transducer 140 can be positioned between the membranes 110, 120 and between the struts 151a, 151b as illustrated in the central portion 104 of the end effector 100.

The piezoelectric transducers 140 can include lead zirconate titanate (PZT). The piezoelectric transducers 140 can be configured as ultrasound (ultrasonic) transducers. An ultrasonic transducer 140 can be configured to transmit acoustic radiation force impulses (ARFIs) to tissue. When the transducer 140 is positioned under an electrode 130a, 130b, the transducer 140 can be coupled to the electrode 130a, 130b to transmit ARFIs through the electrode 130a, 130b to tissue. The impulses displace the tissue by an amount that depends on the elasticity of the tissue, and the transducer 140 can be configured to measure the displacement of the tissue. Because ablated and non-ablated tissues have different elasticities, the different displacements enable the end effector to identify areas of tissue that have been ablated.

In some examples, at least a portion of the electrodes 130a, 130b on the first surface 101a and/or the second surface 101b can be configured to ablate tissue. Having the ultrasonic transducer 140 close to the site of ablation can facilitate monitoring of the ablation. Further, lower ultrasonic energies may be used, while still achieving comparable results to those of external transducers. Examples presented herein can include features disclosed in U.S. 10,492,854, incorporated by reference herein and attached in the Appendix of priority Patent Application No. U.S. 63/400,184, to include an ablation element which is configured to perform ablation of tissue and an ultrasonic transducer, located in proximity to the ablation element, which is configured to transmit ARFIs to the tissue, and to measure a displacement of the tissue in response to the ARFIs so as to monitor the ablation of the tissue.

In some examples, at least a portion of the electrodes 130a, 130b on the first surface 101a and/or the second surface 101b can be configured to map tissue. Having the ultrasonic transducer 140 close to the mapping electrodes 130a, 130b can supplement the mapping signals to increase confidence on the boundaries of an ablated area when mapping in the vicinity of the ablated area.

Figure 1C illustrates a cross-section of the end effector 100 in Figure 1B as sectioned along the longitudinal axis A-A in the right portion 103b of the end effector 100. In this sectional view, it can be seen that underlying strut 151b is between the first membrane 110 and second membrane 120. The first electrodes 132a are disposed on the first membrane while the second electrodes 132b are disposed on the second membrane 120 such that each first electrode 132a on its first membrane 110 has its counterpart second electrode 132b disposed on the second membrane 120 so that each pair of electrodes (e.g., 131a:131b;132a:132b; 133a:133b) are substantially centered on their centerline CL axes. It is noted that the scope of the invention does not require each electrode pair to have their centerline coincident with each other. That is, the center line CLa of one electrode on one membrane to its counterpart centerline CLb for the electrode on the other membrane can be offset to each other, as shown herein Figure 1C. Note the transducer 140 illustrated in Figure 1B is omitted in Figure 1C for the sake of simplifying the illustration.

Figure 1D is a distal end view of the end effector as indicated in Figure 1A. The end effector 100 can have a curvature defining an arcuate path that curves slightly around the longitudinal axis A-A. The longitudinal axis A-A is into the page as the end effector 100 is oriented in the illustration of Figure 1D. The exposed end of the end effector 100 is bowed toward the first orthogonal direction O1 so that the first surface 101a is concave and the second surface 101b is convex. The curvature of the end effector 100 can facilitate collapse of the end effector 100 to fit within a delivery catheter as the end effector 100 is delivered distally through the delivery catheter and as the end effector 100 is retracted proximally into a distal end of delivery catheter.

The end effector 100 can be configured such that when either surface 101a, 101b is pressed to tissue, the surface 101a, 101b conforms to the shape of the tissue. For instance, when either surface 101a, 101b is pressed to a planar surface, the end effector 100 can become planar similar to as illustrated in Figure 1B. The end effector 100 can be moved to conform to a surface solely by manipulation of a shaft of a catheter that includes the end effector. For instance, the shaft of the catheter can be manipulated directly and/or the shaft can be manipulated at least in part through manipulation of a delivery catheter in which the shaft is positioned.

When one of the surfaces 101a, 101b are pressed to tissue, electrodes on the opposite surface (i.e., the surface facing away without contacting tissues) can function as reference electrodes. For electrodes having a counterpart opposite electrode, an electrode that is opposite of an electrode in contact with tissue can be used as a reference electrode for the electrode in contact with tissue. Further, a pair of electrodes on the first surface 101a can function as a bipolar pair while an electrode or a pair of electrodes on the second surface 101b can function as reference electrode(s) for the pair of electrodes on the first surface 101a (and vice versa). A pair of electrodes on the second surface 101b that are opposite the pair of bipolar electrodes on the first surface 101a can be utilized to reduce noise in the electrical signal from the pair of bipolar electrodes on the first surface 101a.

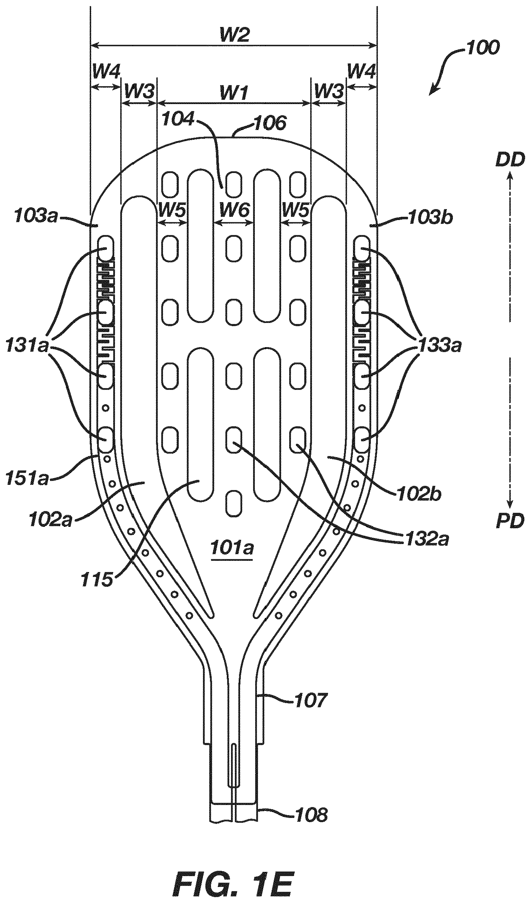

Figure 1E is a planar view of the first side 101a of the example end effector 100. The central portion 104 of the end effector 100 can have a width W1 that is approximately equal to an inner diameter of a delivery catheter through which the end effector 100 is delivered during treatment. The end effector 100 can have a total width W2. The end effector 100 can be configured to bend toward the longitudinal axis A-A at the longitudinal openings 102a, 102b so that the left portion 103a and the right portion 103b fold over the central portion 104 during delivery of the end effector 100 through a delivery catheter.

The longitudinal openings can have a width W3 to provide a desired spacing between electrodes 131a, 132a, 133a in the left portion 103a, right portion 103b, and central portion 104 of the end effector 100. Longitudinal portions of the end effector can have widths W4, W5, W6 to a desired spacing between the electrodes 131a, 132a, 133a and an edge of the surface 101a. Electrodes 131a, 132a, 133a can be positioned between longitudinal openings 102a, 102b, 115 to provide a predetermined width between an edge of an electrode and the respective opening. Electrodes in one longitudinal region can have a different edge spacing than another longitudinal region. For instance, the width W4 of the left and right portions 103a, 103b can be less than a central width W6 of the central portion 104 so that electrodes 131a, 133a positioned in the left and right portions 103a, 103b have a shorter edge spacing than electrodes 132a centered in the central region 104.

The distal end 106 of the end effector 100 can be shaped as a single arc that extends across the width W2 of the end effector. The membranes 110, 120 can extend over the distal ends of the struts 151a, 151b and across the distal end 106 of the end effector 100.

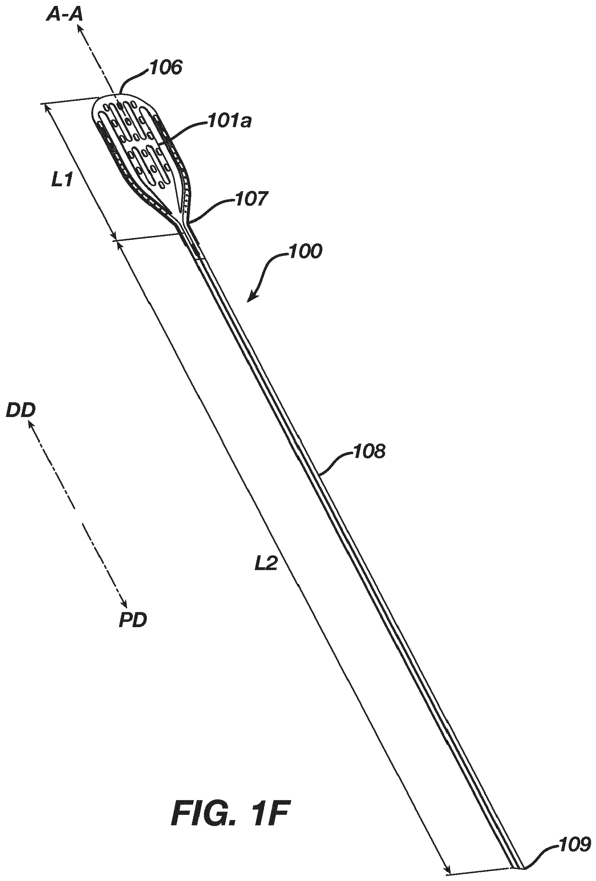

Figure 1F is an isometric view of the first side 101a of the example end effector 100 zoomed out to show a length L2 of the proximal extension 108 of the end effector 100 that can be positioned within a catheter shaft 90 (see Figure 1A). The length L2 of the proximal extension 108 can be measured from a proximal portion 107 of the end effector corresponding to a distal end of a catheter shaft 90 (see Figure 1A) to a proximal end 109 of the proximal extension 108. One or both of the membranes 110, 120 can be positioned in the proximal extension 108 of the end effector and can include electrically conductive traces that are in electrical contact with the electrodes 130a, 130b. The proximal extension 108 can further include additional electrically conductive traces to make electrical contact to the piezoelectric transducers 140, other sensors disclosed herein, and other end effector features as understood by a person skilled in the pertinent art.

In one example, proximal extension 108 can have a length L2 of about 10 centimeters (cm) that extends through a flexible distal portion of the catheter shaft 90. The proximal extension 108 can include solder pads so that wires can be attached to the end effector 100 to provide electrically conductive paths to the electrical components of the end effector 100. In this example, the proximal end 109 of the proximal extension 108 is surrounded by the catheter shaft 90 (see Figure 1A).

In another example, the proximal extension 108 can extend through the entirety of the catheter shaft 90. Typical catheter shafts have an insertion length of between about 60 cm and about 130 cm, with 115 cm being a common standard insertion length. The proximal extension 108 can therefore have length that is about as long as the catheter shaft insertion length, possibly a few centimeters longer so that the proximal end 109 of the proximal extension 108 is near, or slightly distal to, the proximal end of the catheter shaft.

The catheter can include a handle (not illustrated) as understood by a person skilled in the pertinent art. The proximal extension 108 can extend into the handle. In one example, the proximal extension can include a proximal portion configured for mounting a control circuit on the proximal extension and within the control handle (not illustrated). The control circuit portion of the proximal extension 108 can have a larger width than the portion of the proximal extension 108 that traverses the catheter shaft. The control circuit can be configured similar to flex circuits in compact electronics as understood by a person skilled in the pertinent art.

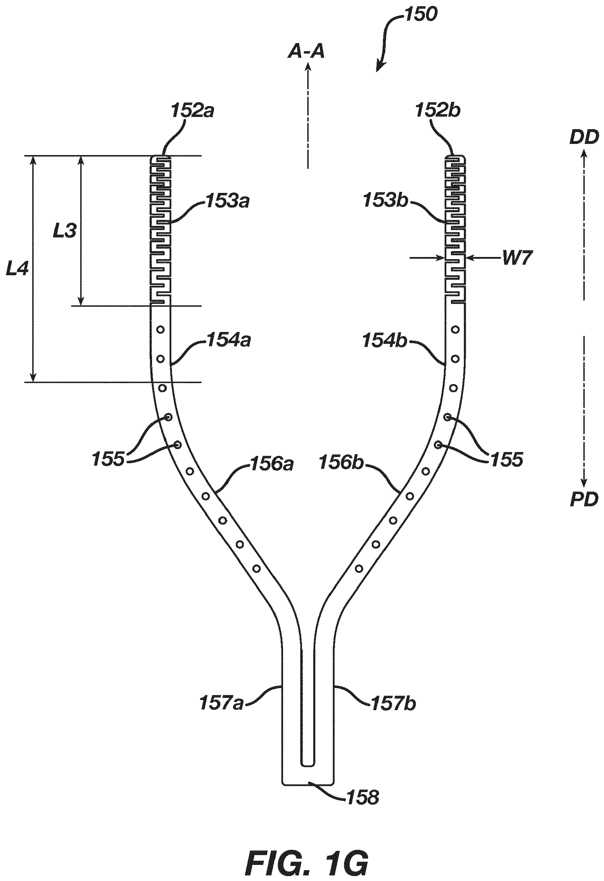

Figure 1G is an illustration of the frame 150 of the end effector 100. The frame 150 includes a left strut 151a and a right strut 151b that are symmetric about the longitudinal axis A-A. The width W7 of each of the frame struts 151a, 151b is approximately equal through the length of the respective strut 151a, 151b.

The frame 150 terminates distally in distal ends 152a, 152b of the struts 151a, 151b. The struts 151a, 151b respectively include a longitudinal portion 154a, 154b through which the strut 151a, 151b extends along the longitudinal axis A-A and substantially parallel to the longitudinal axis A-A. The struts 151a, 151b each include an angled portion 156a, 156b that extends along the longitudinal axis and is angled centrally toward the longitudinal axis A-A in the proximal direction (PD). The struts 151a, 151b each include a proximal portion 157a, 157b that extends along the longitudinal axis A-A and substantially parallel to the longitudinal axis. The proximal portions 157a, 157b of the struts 151a, 151b are joined by a proximal connecting portion 158 of the frame 150. Preferably, the proximal portions 157a, 157b and proximal connecting portion 158 are shaped to be positioned within a catheter shaft of an assembled catheter.

The struts 151a, 151b each include a distal flexible region 153a, 153b over length L3 extending in the proximal direction (PD) from the respective distal end 152a, 152b. The flexible region 153a, 153b has a flexibility gradient over the length L3 of the flexible region such that the strut 151a, 151b becomes more flexible toward the distal end 152a, 152b, in the distal direction (DD). The flexible region 153a, 153b has a length L3 that is at least half of the total length L4 of the longitudinal portion 154a, 154b. The flexible region includes a cutout pattern that is graduated to achieve the flexibility gradient. The cutout pattern is a zig-zag or serpentine pattern.

The frame 150 further includes circular cutouts 155 positioned proximal of the flexible region to achieve moderate flexibility. The circular cutouts 155 are positioned through a proximal portion of the longitudinal portions 154a, 154b of the struts 151a, 151b and through a majority of the angled portions 156a, 156b of the struts 151a, 151b. The proximal portions 156a, 156b and connecting portion 158 of the frame 150 lack cutouts.

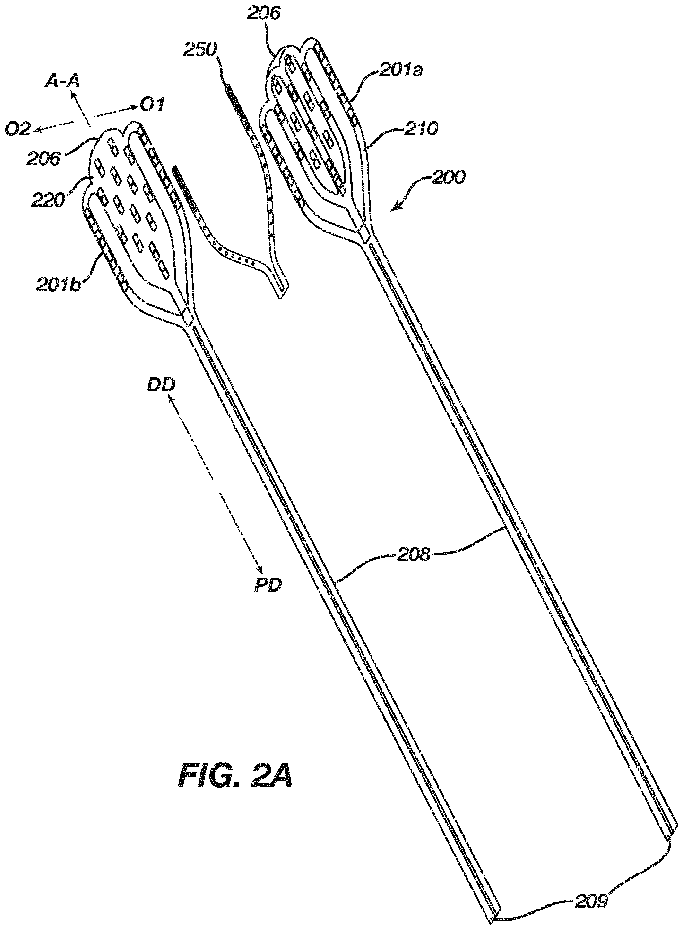

Figures 2A through 2E are illustrations of various view and components of a second example end effector 200.

Figure 2A is an isometric exploded view of the end effector 200. Similar to the end effector 100 illustrated in Figures 1A through 1G, the end effector 200 illustrated in Figures 2A through 2E has a first surface 201a, an opposite second surface 201b, a first membrane 210, a second membrane 220, and a frame 250 between the membranes 210, 220. The end effector 200 illustrated in Figures 2A through 2E has a distal end 206 and a proximal end 209 and is oriented similarly as the end effector 100 illustrated in Figures 1A through 1G with respect to the longitudinal axis A-A, orthogonal directions 01, 02, distal direction (DD), and proximal direction (PD). The end effector 200 can further include a member disposed between the membranes 210, 220 similar to the structural member 142 illustrated in Figure 1B. The end effector 200 includes a proximal extension 208 that can be configured similarly to the proximal extension 108 illustrated in Figure 1F. In a preferred embodiment, the frame 250 or structural member 142 can be selected from a group of materials such as polymer, metallic, semi-metallic, rigid, elastic or super elastic material and combinations hereof. The membranes 210 and 220 can be selected from group of insulator, di-electric, insulating di-electric such as polyimide, polyester, fluorocarbon films, aramid, composites and combinations hereof.

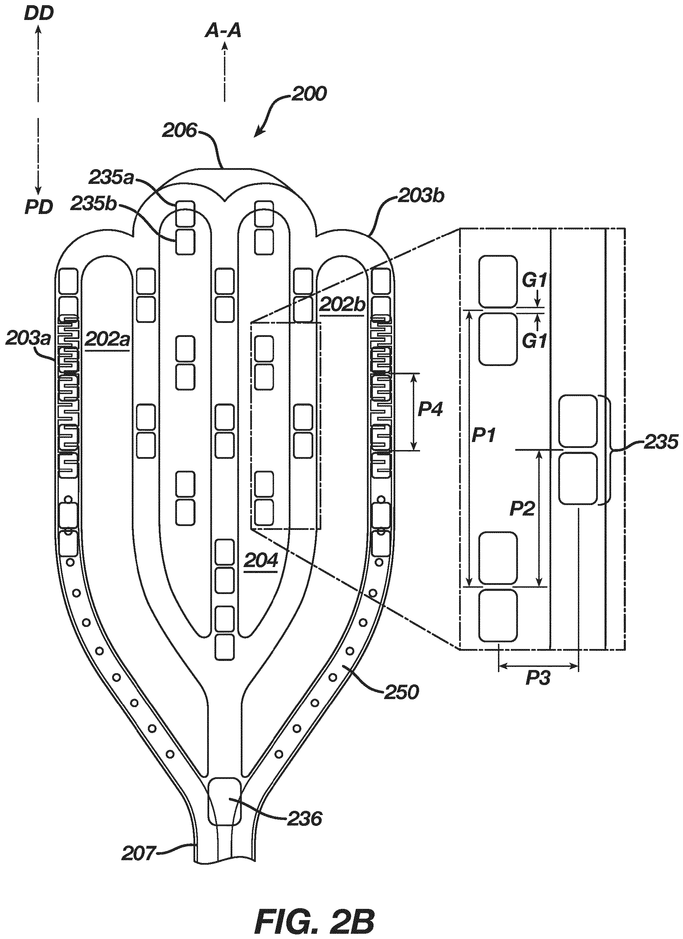

Figure 2B is a planar view of the first side 201a of the end effector 200. The end effector 200 includes longitudinal openings 202a, 202b separating a left portion 203a, a central portion 204, and a right portion 203b of the end effector 200 similar to the longitudinal openings 102a, 102b and end effector portions 103a, 104, 103b of the end effector 100 illustrated in Figures 1A through 1G. The frame 250 of the end effector 200 can be configured similarly to the frame 150 of the end effector 100 illustrated in Figures 1A through 1G.

The end effector 200 includes electrode pairs 235a, 235b on the first side 201a that can be configured to function as a bipolar pair 235. The bipolar pairs 235 can include a gap distance G1 between electrodes 235a, 235b in the electrode pair 235 that is an edge-to-edge spacing. The gap distance can measure about 150 micrometers. Referring to the center portion 204 of the end effector 200, the bipolar pairs can be spaced with a longitudinal pitch P1 between longitudinally aligned bipolar pairs 235 that is measured center-to-center. The bipolar pairs 235 can be staggered with a longitudinal pitch P2 and a lateral pitch P3 between staggered pairs 235. The longitudinal pitch P2 between staggered pairs can be about half of the longitudinal pitch P1 between longitudinally aligned pairs 235. The electrode pairs 235 in the left portion 203a and the right portion 203b of the end effector 200 can have a longitudinal pitch P4 that is approximately equal to the longitudinal pitch P2 between the staggered pairs of the central portion 204 and can measure about 2.5 millimeters.

The end effector further includes a reference electrode 236 positioned at a proximal portion 207 of the end effector 200. The reference electrode 236 can be positioned to contact fluid during treatment, but positioned to be inhibited, by the catheter shaft, from contacting tissue during treatment. The reference electrode 236 can have a larger surface area compared to a single electrode 235a, 235b of an electrode pair 235.

The end effector 200 can include three arcs across the distal end 206 of the end effector. A first arc can extend distally from the left portion 203a of the end effector, curve toward the longitudinal axis A-A, and curve in the proximal direction (PD) to the central portion 204 of the end effector, concave in the proximal direction (PD). A second arc can curve across the distal end of the central portion 204. A third arc can extend from the central portion 204 to the right portion 203b, concave toward the proximal direction (PD).

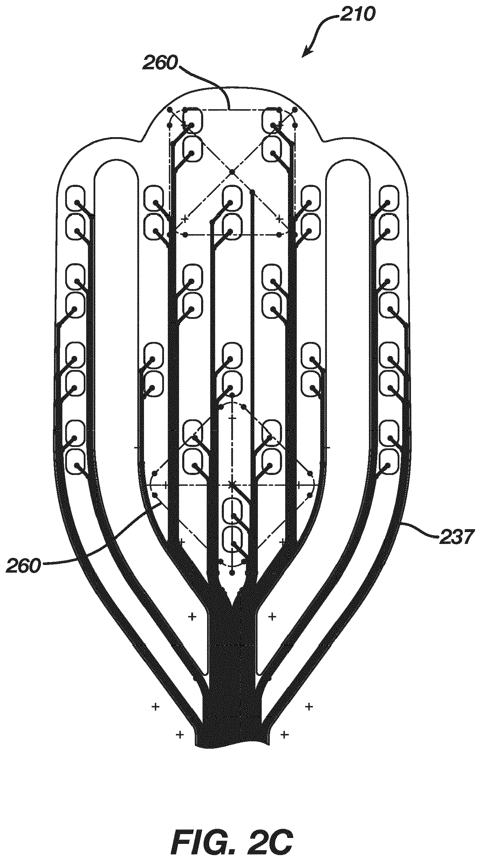

Figure 2C illustrates aspects of the first membrane 210 including electrical traces 237. Because the membranes 210, 220 can include flex circuits, the end effector 200 can include components, structures, and features compatible with flex circuit technology. For instance, the end effector can include a sensor 260 or other component in one or more flex circuit layers under the top, electrode layer. Figure 2C indicates two approximately square shaped regions 260 that are examples of suitable locations for such a feature. For instance, the end effector 200 can include planar spirals in these regions 260 that can function as single axis sensors (SAS). Each SAS can have dimensions of about 4 mm by 4 mm, or an area of about 16 mm². The sensor 260 can include a double axis sensor and need not be planar.

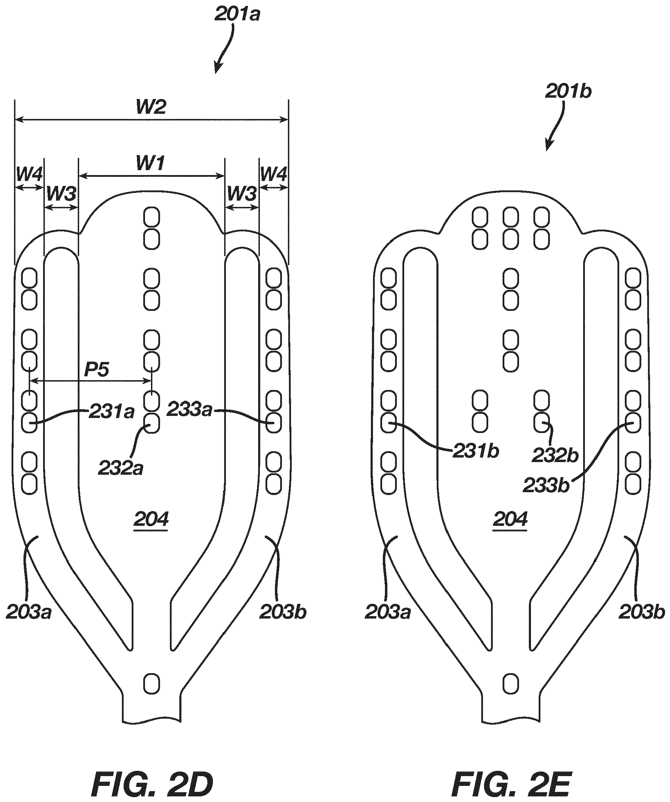

Figures 2D and 2E are illustrations of the first surface 201a and the second surface 201b in an example in which the electrode pattern differs on the first surface 201a compared to the second surface 201b. Some of the electrodes on each surface 201a, 201b have corresponding opposite electrodes on the other surface, and some do not. The electrode pattern on each surface 201a, 201b can be varied to meet design considerations for a particular end effector.

As illustrated in Figure 2D, the end effector 200 can have widths W1, W2, W3, W4 similar to those of the end effector 100 illustrated in Figure 1E. Electrodes 231a, 233a in left and right portions 203a, 203b of the end effector 200 can be separated from centrally spaced electrodes 232a in the central portion of the end effector 200 by a lateral pitch P5 of about 5 mm.

As illustrated in Figure 2E, the electrodes 231b, 233b on the left and right portions 203a, 203b of the second surface 201b have corresponding opposite electrodes 231a, 233a on the first surface 201a, however do not match every pair on the first surface 201a. In the central portion 204, two electrode pairs 232b have corresponding opposite electrodes, and for the other two pairs 232a on the first surface 201a, the second surface 201b has two pairs 232b spaced laterally on either side of the respective pair on the first surface 201a.

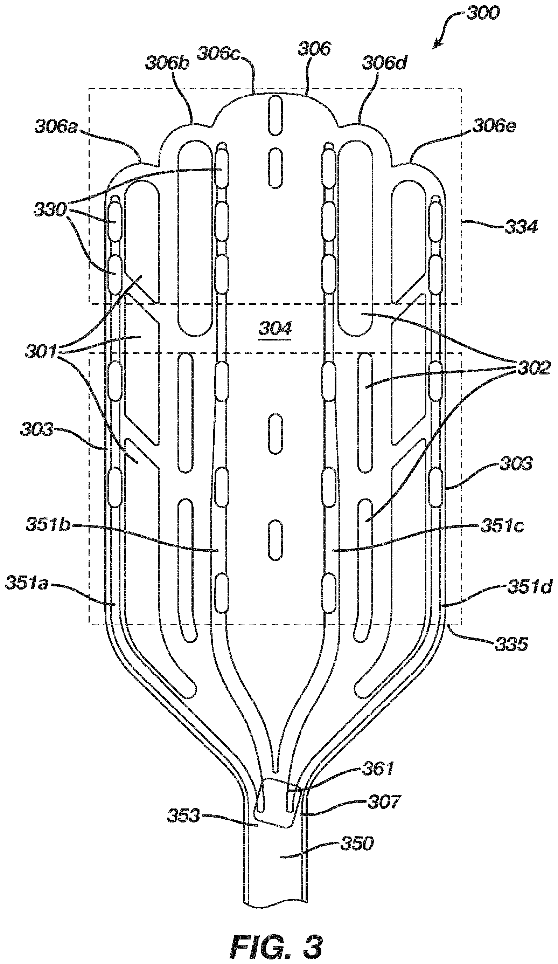

Figure 3 is an illustration of planar view of a third example end effector 300. The end effector 300 includes a frame 350 having four distally extending struts 351a, 351b, 351c, 351d and a proximal strut 353. The distally extending struts 351a, 351b, 351c, 351d can be configured similarly to struts 151a, 151b illustrated in Figure 1G. The end effector 300 includes longitudinal openings 301, 302 that separate the end effector into left and right portions 303 and a central portion 304. A portion of surface electrodes 330 are positioned over struts 351a, 351b, 351c, 351d of the frame 350, and a portion of surface electrodes 330 are positioned between struts 351b, 351c. The end effector 300 has five arcs 306a, 306b, 306c, 306d, 306e shaping a distal end 306 of the end effector 300. The end effector 300 can be otherwise configured with compatible features of other end effectors disclosed herein, alternatives thereto, and variations thereof.

The electrodes 330 are spaced in a high density region 334 near the distal end 306 of the end effector 300 and a lower density region 335 proximal of the high density region 334. As illustrated, the high density region 334 has the same number of electrodes 330 as the low density region 335 within a smaller surface area. The electrodes 330 in the high density region 334 have the same longitudinal pitch as in the low density region 335 and a shorter longitudinal pitch in the high density region 334 compared to the low density region 335.

The end effector 300 includes an off-axis SAS 361 at a proximal portion 307 of the end effector 300. The off-axis SAS 361 can be oriented at an angle to the plane of the end effector 300, or at an angle to the plane of the page of the illustration. For the sake of illustration, the SAS 361 is shown tilted in the plane of the page; however, it is to be understood that the tilt of the SAS 361 is into or out of the plane of the page in the assembled end effector 300. The end effector 300 can further include one or more SAS in the plane of the end effector 300 similar to as illustrated in Figure 2C.

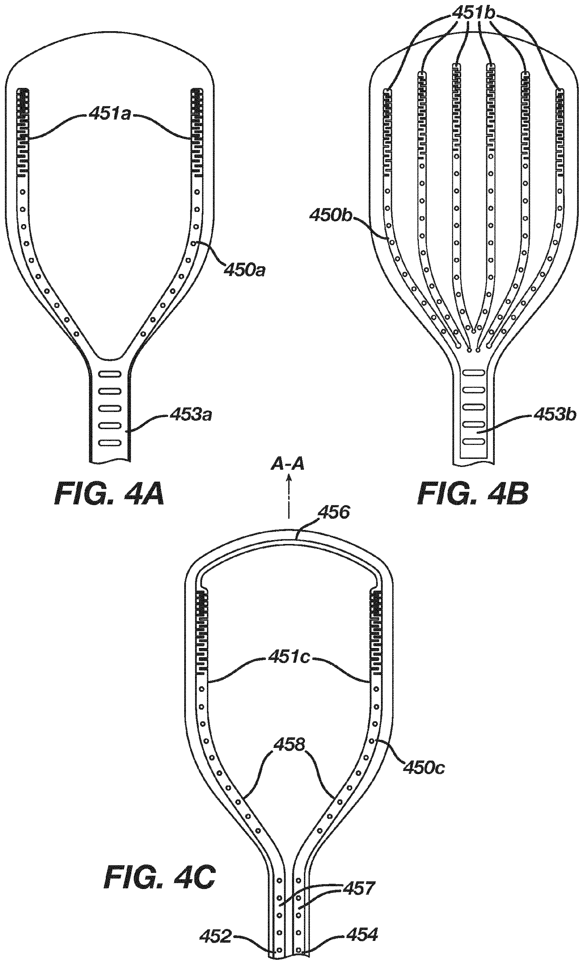

Figures 4A through 4C are illustrations of example frame configurations. Figure A illustrates a frame 450a having two distally extending struts 451a configured similarly to struts 151a, 151b illustrated in Figure 1G with a proximal strut 453a configured similarly to the proximal strut 353 illustrated in Figure 3.

Figure 4B illustrates a frame 450b configured similarly to the frame 450a illustrated in Figure 4A with additional distally extending struts 451b. The frame 450a as illustrated includes six distally extending struts 451b extending distally from a proximal strut 453b. The frame 450b can be modified to include three, four, or five distally extending struts 451b.

Figure 4C illustrates a frame 450c having a looped strut including longitudinal proximal ends 452, 454, proximal segments 457 extending distally from the proximal ends 457, angled segments 458 that angle away from the longitudinal axis A-A distally, longitudinal segments 451c that extend along the longitudinal axis A-A distally from the angled segments 458, and a connector segment 456 that extends across a distal end of the frame across the longitudinal axis A-A. The looped strut forms a continuous, non-branching path from a first proximal end 452 to a second proximal end 454. The looped strut can be positioned near a perimeter of the exposed end of the end effector. The longitudinal segments 458 can include a flexibility gradient as disclosed elsewhere herein.

The looped strut can be configured to function as a navigation sensor loop. Electrical conductors can be attached at the proximal ends 452, 454 of the looped strut and extend through a catheter shaft to a handle to be connected to a catheter control system.

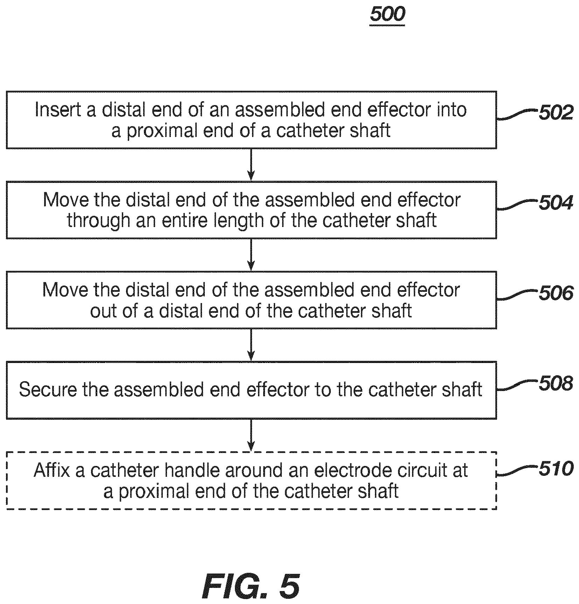

Figure 5 is a flow diagram of a method 500 for constructing a catheter. The end effectors 100, 200, 300, illustrated in Figures 1A through 3 and variations thereof, including variations having frames configured similarly to frames 450a, 450b, 450c illustrated in Figures 4A through 4C, can be collapsible to a sufficiently small diameter such that a distal portion of an assembled end effector can be inserted into a proximal end of a catheter shaft, fed through the entire length of the catheter shaft, and positioned distal of the distal end of the catheter shaft. This can provide an alternative method for construction of multi-electrode catheters, which typically require that the end effector be inserted at a distal end of the catheter shaft because the distal portion of the end effector is too large to traverse the catheter shaft.

At step 502, a distal end of an assembled end effector is inserted into a proximal end of a catheter shaft.

At step 504, the distal end of the assembled end effector is moved through an entire length of the catheter shaft.

At step 506, the distal end of the end effector is moved out of a distal end of the catheter shaft.

At step 508, the assembled end effector can be secured to the catheter shaft. Once secured, the end effector can be positioned in relation to the shaft similar to as illustrated in Figure 1A.

At optional step 510, a catheter handle can be affixed around an electronic circuit at a proximal end of the catheter shaft. Because the end effector is inserted at a proximal end of the catheter shaft, rather than the distal end, the proximal portion of the end effector can have dimensions that are too large to be inserted into the catheter shaft. The allows for an electronic circuit to be pre-attached to the end effector. This can provide an alternative method of construction of multi-electrode catheters which require that electronics in the handle be attached after conductors connected to the distal portion of the end effector are fed through the catheter shaft.

The assembled end effector can have various configurations to facilitate the method 500. The assembled end effector can include a flex circuit with conductors that extend through the entirety of the catheter shaft, or the assembled end effector can include wires through at least a portion of the catheter shaft. The electronic circuit at the proximal end of the assembled end effector can be rigid or flexible. The electronic circuit can include various components such as surface mounted components, integrated circuits, electrical traces, etc. as understood by a person skilled in the pertinent art.

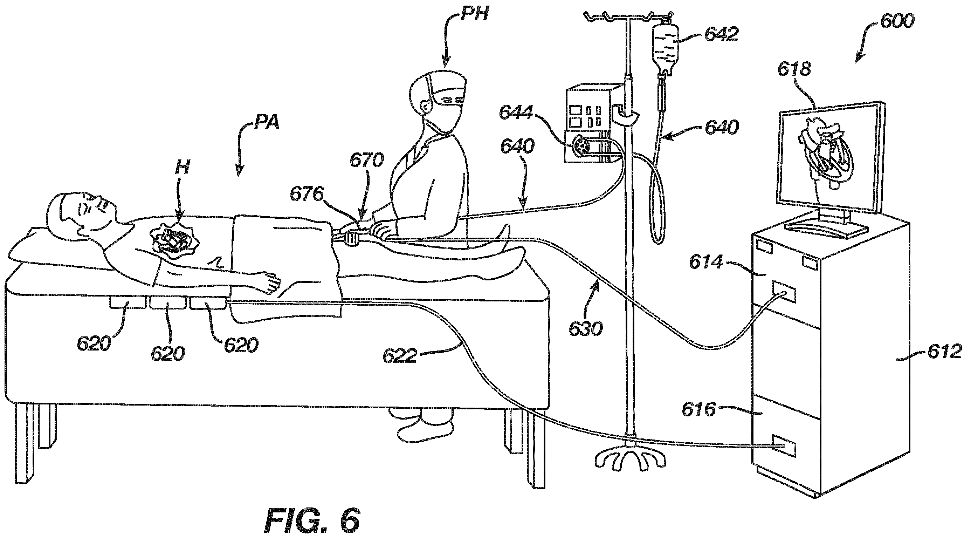

Figure 6 is an illustration of a medical procedure utilizing a catheter 670 and a catheter control system 600. The catheter 670 can include an end effector configured similarly to an example end effector disclosed herein, a variation thereof, or an alternative thereto as understood by a person skilled in the pertinent art. The control system 600 can include a console 612 and a display 618. The physician PH can manipulate a handle 676 of the catheter 670 to position the end effector within or near a heart H of a patient PA.

The catheter 670 is coupled to the control system 600 via a cable 630 the includes electrical conductors to carry electrical signals between the catheter 670 and control system and/or provide power to the catheter 670. Alternatively, the catheter 670 can communicate wirelessly to the control system 600 and/or the catheter 670 can have a power source separate from the control system 600. The console 612 can include a catheter driver module 614 configured to communicate with the catheter 670. The catheter driver module 614 can include one or more processors and non-transitory computer readable medium (memory) storing instructions that can be executed by the processor(s). The memory can include instructions to cause the system 600 to receive a mapping electrical signal from mapping electrodes of the catheter 670 that are in contact with tissue and receive a reference electrical signal from a reference electrode of the catheter 670 that are facing away from the tissue on an opposite side of the end effector. The mapping electrical signal is preferably a bipolar signal from two electrodes that are in contact with tissue; however the mapping electrical signal may be unipolar from one electrode that is in contact with tissue. The instructions can cause the system to receive multiple mapping electrical signals that may include bipolar and/or unipolar signals. The reference electrode can be opposite a mapping electrode. The instructions can cause the system to receive one or more reference electrical signal(s) from multiple reference electrodes that are not in contact with tissue. At least a portion of the reference electrodes can be opposite the mapping electrodes. The instructions can reduce noise in the mapping electrical signal through comparison to the reference electrical signal. The instructions can cause the system to receive a bipolar mapping electrical signal from a first pair of bipolar electrodes of the end effector in contact with tissue and receive one or more reference electrode signal(s) from a second pair of reference electrodes disposed opposite the first pair of bipolar electrodes and facing away from tissue.

The instructions can further cause the system to utilize tomography electrodes that are on the side of the end effector in contact with tissue to perform impedance tomography of tissue. The instructions can be executed to apply an alternating current to a first plurality of tomography electrodes disposed on the first side of the end effector, receive equi-potentials of a second plurality of tomography electrodes paired with the first plurality of tomography electrodes and disposed on the first side of the end effector, generate a tomographic image of the tissue based on the equi-potentials, and display the tomographic image on the display 618.

The instructions can further cause the system to receive a navigation signal from a SAS coil disposed in a plane that is between the mapping electrode(s) and the reference electrode(s). The instructions can further cause the system to receive a navigation signal from a SAS that is at an off-angle plane that is at an angle to an end effector plane.

Magnetic field generators 620 are positioned under the patient PA and are also coupled to the control system 600 via a cable 622. The control system 600 can provide power and/or control signals to the field generators 620. Alternatively, communication between the field generators 620 and control system 600 can be wireless and/or the field generators can have a power source separate from the control system 600. The console 612 includes a module 616 configured to control the magnetic field generators 620. The module 616 can include one or more processors and memory with instructions thereon that can be executed by the one or more processors to cause the system 600 to provide a magnetic field through the patient PA by utilizing the magnetic field generators 620. The magnetic field can be sensed by navigation sensors, including SAS, to determine a location of the end effector of the catheter 670 within the patient PA.

The catheter 670 can be coupled with a fluid source 642 via a conduit 640 and a pump 644. The fluid source 642 can provide irrigation for a catheter 670 having irrigation capabilities in a medical procedure utilizing irrigation.

The console 612 can further include a module configured for impedance-based catheter navigation. Conductive body patches can be applied to the patient PA, and the console 612can be configured to measure impedance between body patches and one or more electrodes of the end effector of the catheter 670. The console 612 and the catheter 670 can be configured for magnetic-cased position sensing and/or impedance-based position sensing as disclosed for example in U.S. 7,848,789, incorporated by reference herein and attached in the Appendix of priority Patent Application No. U.S. 63/400,184, or as otherwise understood by a person skilled in the pertinent art. The console 612 can include one or more processors and a memory with instructions thereon that can be executed by the processor(s) to cause the control system 600 to determine a positioned of the end effector based at least in part on an impedance between an electrode of the end effector and a body patch electrode.

The console 612 can further be configured with a generator to provide an electrical signal to one or more electrodes of the end effector to perform ablation. Energy produced by the ablation energy generator may include, but is not limited to, radiofrequency (RF) energy or pulsed-field ablation (PFA) energy, including monopolar or bipolar high-voltage DC pulses as may be used to effect irreversible electroporation (IRE), or combinations thereof. To achieve IRE, short pulses of high voltage electrical signals are delivered to tissues; the electrical signals generate an unrecoverable permeabilization of cell membranes. Delivery of IRE energy to tissues using multi-electrode catheters was previously proposed in the patent literature. Examples of systems and devices configured for IRE ablation are disclosed in U.S. Patent Pub. No. 2021/0169550A1, 2021/0169567A1, 2021/0169568A1, 2021/0161592A1, 2021/0196372A1, 2021/0177503A1, and 2021/0186604A1, each of which are incorporated herein by reference.

One commercial product embodying elements of the system 600 is available as the CARTO^{™} 3 System, available from Biosense Webster, Inc.

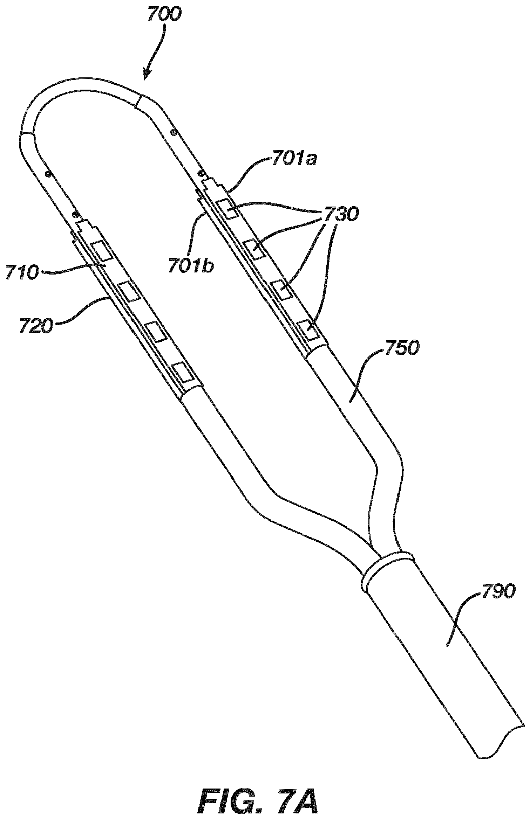

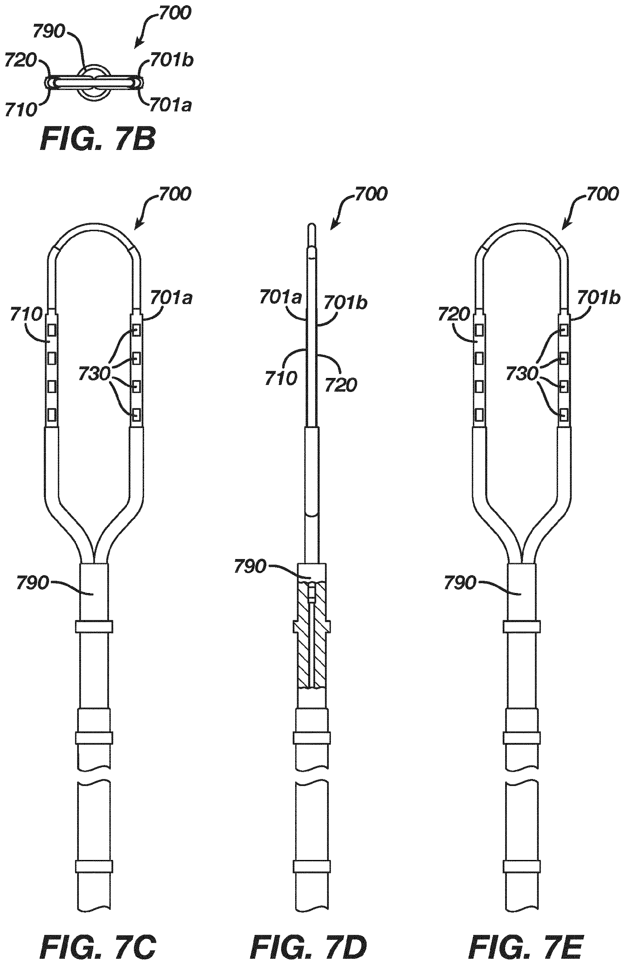

Figures 7A through 7E illustrate a catheter 700 including another example end effector having two sides 701a, 701b with a first membrane 710 on the first side 701a and a second membrane 720 on the second side 701b and electrodes 730 on each side. The end effector includes a single loop extending distally from a catheter shaft 790. End effectors illustrated elsewhere herein can be affixed to a catheter shaft similar to the shaft 790 illustrated in Figures 7A through 7E, variations thereof, and alternatives thereto as understood by a person skilled in the pertinent art.

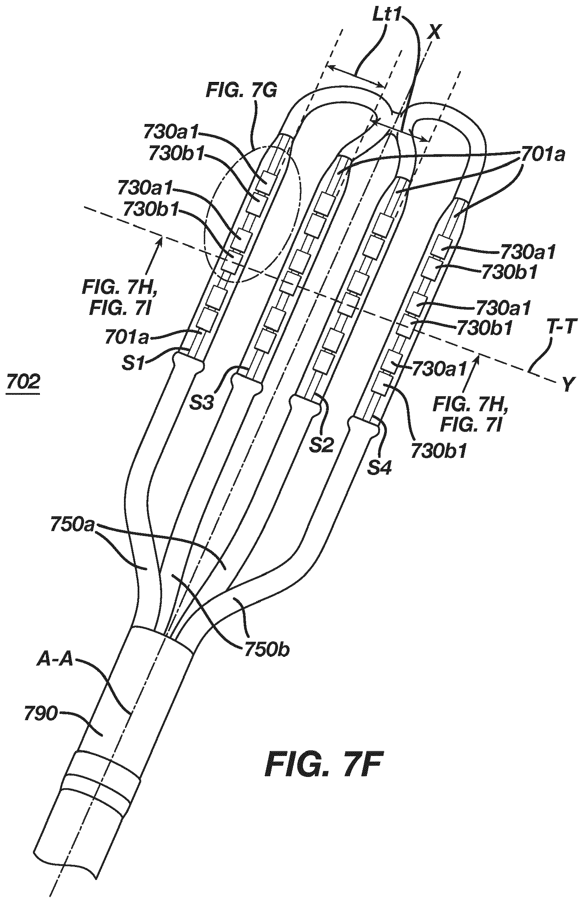

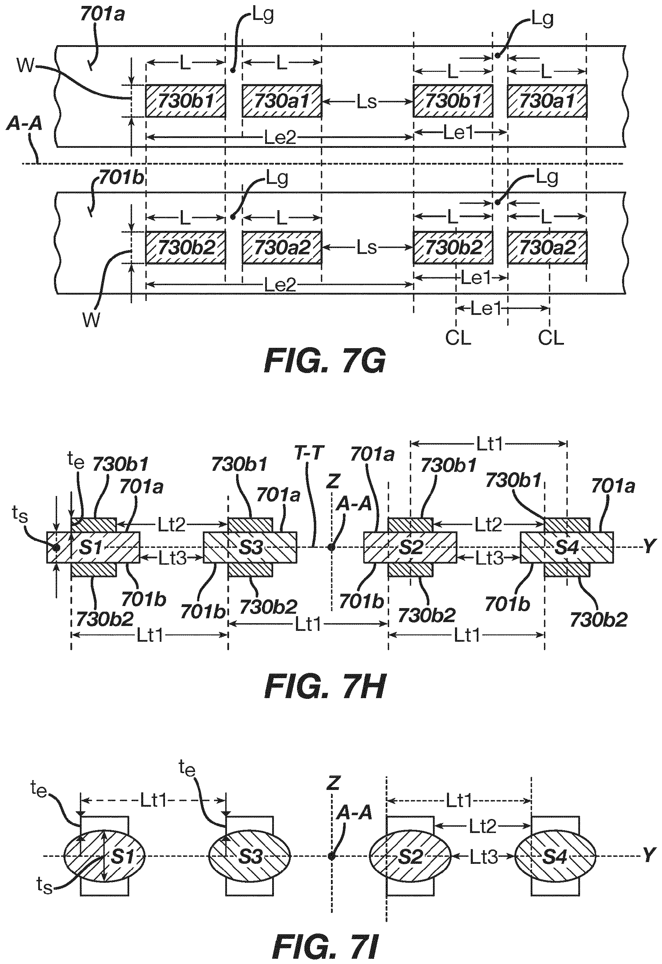

Figure 7F shows a plural loop version of single loop embodiment of Figure 7A in which at least two loops 750a and 750 overlap each other near the longitudinal axis A-A. Loop 750a may have a first segment S1 and a second segment S2 that extend alongside each other. Loop 750b may have a third segment S3 and a fourth segment S4 that extend alongside each other. This is shown in sectional view of Figure 7H in which each segment (S1, S2, S3, S4) in Figure 7F has two generally opposed outer surfaces: a first outer surface (surface 701a in Figure 7G) presenting a four closely spaced bipole electrode pairs (730a and 730b forming a pair) with the other opposite second outer surface (701b in Figure 7G) presenting counterpart and essentially identical bipole electrodes for a total of 12 bipole electrode pairs (or 24 individual electrodes) on the top surface and 12 bipole electrode pairs (or 24 individual electrodes) on the bottom surface (not shown) in Figure 7F. The first and second outer surfaces 701a and 701b can be substantially parallel to each other in one arrangement as shown in Figures 7H and 7I. It is further noted that segment S1 may be connected to segment S2 to form a distal loop for loop 750a and segments S3 and S4 may be connected to form loop 750b.

In Figure 7G, a first outer surface 701a of segment S1 (of Figure 7F) is shown with the second outer surface 701b for ease of understanding of the double-sided arrangement of closely-spaced bipole electrodes. Specifically, with reference to the first outer surface 701a of Figure 7G, each pair of bipole electrodes (730a1,730b1) has one electrode 730a1 spaced apart from its partner electrode 730b1 (of the same closely spaced pair) with a longitudinal gap Lg of any value from about 0.1 mm to about 1 mm and preferably about 0.25 mm. In the example of Figure 7G, electrode 730a1 is spaced apart from its partner electrode 730b as measured from corresponding edge of electrode 730a to edge of electrode 730b for a distance **Le1** from about 0.3mm to about 2 mm and preferably about 0.85 mm. Where the electrodes in each electrode pair is identical, the distance **Le1** can be measured from the center of one electrode to the other electrode of the same pair. The electrode 730b1 of one bipole pair is spaced over a length Le2 (measured from edge to edge along the longitudinal axis A-A) to corresponding electrode 730b1 of another bipole pair where Le2 can be from about 1.5 mm to approximately 4mm and preferably about 2.4mm.

Additionally, an edge of one electrode (730b1) in one pair (730a1 and 730b1 on the right side of Figure 7G) can be spaced apart from an edge of another electrode (730a1) of an adjacent pair (730b1 and 730a1 on the left side of Figure 7G) over a distance **Ls** of any value from approximately 0.5 mm to 2 mm and preferably about 0.95 mm. Each electrode 730a1, 730a2, 730b1, or 730b2 may have an exposed surface area (for physical contact with tissue) from about 0.04 mm-squared to about 1 mm squared. In Figure 7G, length L can have any value from approximately 0.1 mm to approximately 1 mm and a width of any value from approximately 0.1 mm to approximately 1 mm.

While the surface area of the electrode is shown in Figure 7G as rectangular, the surface area can be square (linear). Where the perimeter is square, such perimeter can have its sides as any value from approximately 0.1 mm to approximately 1 mm. As well, circular, ovoid or a perimeter being non-linear can be utilized as long as the non-linear perimeter can be approximated to the aforementioned surface areas for the electrode. In the preferred embodiments, the surface area of each electrode is any value from about 0.04 mm-squared to about 1 mm-squared and preferably about 0.3 mm-squared.

With reference to the second and opposite outer surface 701b in Figure 7G, the same arrangement in dimensions and measurements for each closely-spaced bipole pair 730a1 and 730b1 are also applicable to the two counterpart bipole pairs 730a2 and 730b2 shown in Figure 7G.

With reference to sectional view of Figure 7H which is taken along transverse axis T-T (or y axis in Cartesian datum), it can be seen that segments S1, S2, S3, S4 are spaced along transverse axis T-T (or y-axis) with each segment shown exemplarily as rectangular in cross-section. In this arrangement, each electrode pair on one segment (e.g., S2) is spaced apart to another electrode pair on adjacent segment (e.g., S3 or S4) over a first transverse gap distance **Lt1** (electrode edge to electrode edge) as shown in Figure 7F and more particularly in its cross-sectional view shown in Figure 7H. The electrodes 730b are spaced apart along an axis T-T (y-axis) transverse to the longitudinal axis A-A such that the transverse gap length **Lt1** between adjacent electrodes (as measured edge to edge or electrode center to electrode center) may have any value from approximately 0.5 mm to approximately 4 mm and preferably about 2.4 mm. The electrode 730b1 on one segment (S1) is spaced apart from its electrode edge to a nearest electrode edge of another electrode 730b1 on adjacent segment (S2) over a second transverse gap (**Lt2**) having any value from approximately 0.3mm to approximately 3.8 mm and preferably about 1.9 mm. The nearest edges of adjacent segments (e.g. S1 and S3) may be a third transverse gap **Lt3** being less than the second transverse gap **Lt2.**

In Figure 7H, each segment (S1, S2, S3, S4) may have a thickness ts of any value from approximately 0.3 mm to approximately 1mm and preferably about 0.4 to 0.7 mm as referenced to the z-axis. It is noted that the cross-section of each segment is not limited to the rectangular area shown in Fig. 7H but can take other forms such as a non-linear perimeter (or a combinations of linear and non-linear perimeters) for the surface area such as shown in Fig. 7I as long as such surface area is within the range devised herein. Each electrode 730a1, 730a2, 730b1 or 730b2 may have its thickness te of any value from approximately 0.0001 microns or micrometers to approximately 10 microns and preferably any value from 5 microns to 7 microns as referenced to the z-axis. While the cross-sectional area of the electrode is depicted as rectangular, other variations such as square (linear perimeter), circle or ovoid (non-linear perimeter) or combination of linear and non-linear perimeters are within the scope of the claimed invention.

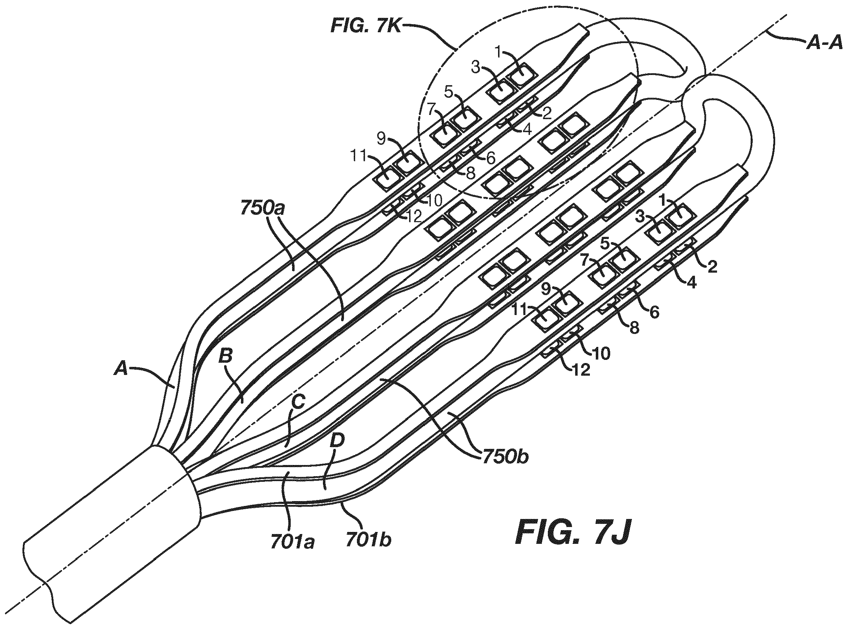

Figure 7J illustrates yet another variation of the end effector illustrated in Figure 7F. In this variation, the first loop 750a or the second loop 750b does not cross over the longitudinal axis A-A. The first loop 750a is composed of spine segments A and B while the second loop 750b is composed of spine segments C and D. On each spine segment A, B, C, D are top substrate 701a and bottom substrate 701b. On each top substrate 701a for each spine, (e.g., A and D in Figure 7J) are disposed three pairs of closely spaced electrodes numbered pair 1:3; pair 5:7; pair 9:11 or as six different electrodes referenced as electrodes 1, 3, 5, 7, 9 and 11. On each opposite, bottom substrate 701b for each spine A,B, C,D are corresponding three pairs of closely spaced electrodes referenced as electrode pairs 2:4; 6:8; and 10:12. These electrode pairs on the opposite surface 701b can also be referred to as electrodes 2, 4, 6, 8 and 10.

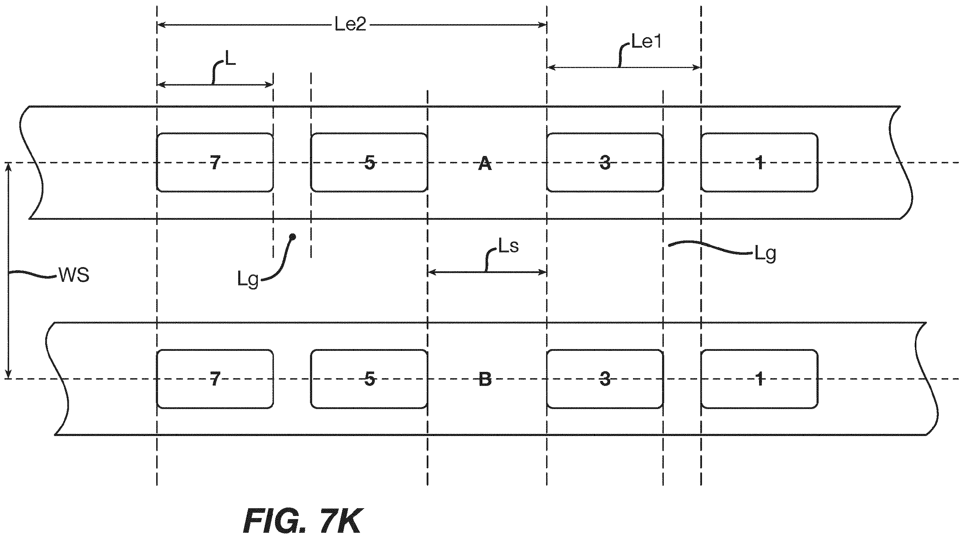

Figure 7K is an illustration of representative dimensions of the closely spaced electrode pairs 1:3 and 5:7 on spine A and its neighbor spine B and corresponding electrode pairs 1:3 and 5:7. The dimensions L for each electrode, with spacing Ls between any two closely spaced electrode pairs (e.g., 7:5 as one pair and 3:1 as a neighboring pair) with distance Le1 from edge to corresponding edge in the same closely spaced electrode pair (e.g., 1:3) with gap length Lg between the facing edges of the same pair (e.g., 1:3) are the same as previously defined elsewhere in this application with the same nomenclatures. In the example of Figure 7J, the center to center distance between neighboring spines A and B is indicated as WS which can range from about 1 mm to about 5 mm. And as used herein, the term "closely spaced electrode pair" means that the two electrodes in a pair are separated by a gap Lg of less than 1 mm and preferably about 0.85mm.

The electrode arrangement with the spines A, B, C, D is represented by the following Table in which the odd electrodes represent a top surface 701a whereas the even numbered electrodes represent electrodes on the opposite bottom surface 701b. It should be noted that the invention is not limited to the nomenclatures of "top surface" and "bottom surface" which could be a "first surface" and a "second surface opposite the first surface". As well, the first surface is not required to be part of the second surface but could be a separate member as shown in Figure 7J.

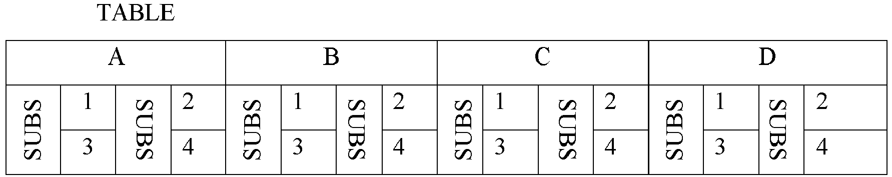

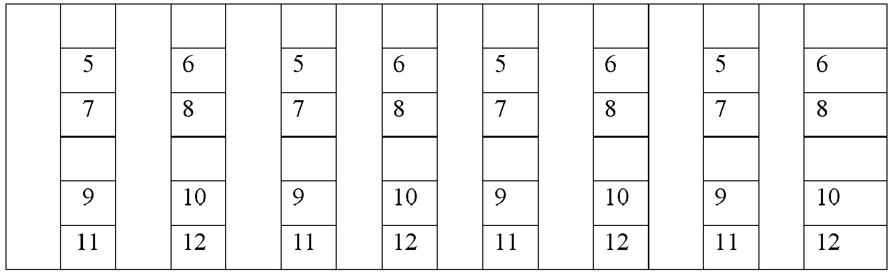

As previously described elsewhere, electrode 1 can act as a reference electrode (being not in contact with tissue) for tissue contact electrode 2 and likewise electrode 3 can act as a reference electrode for tissue contact electrode 4 independently of any other pair of oppositely facing electrodes. This arrangement has the benefit of allowing tissue signals (i.e., ECG signals) collected from the electrodes in tissue contact to subtract noise or far-field signals collected from its counterpart electrode that is not in tissue contact.

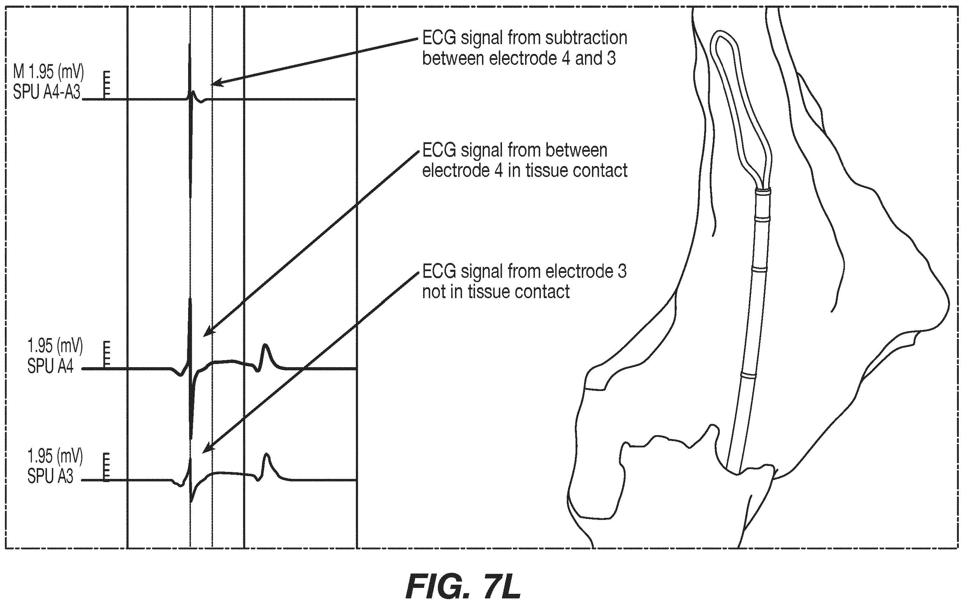

Figure 7L is an illustration of an actual ECG recording of cardiac tissues adjacent an image of the catheter position in a heart as visualized on display 618 of system 600 (Figure 6). As illustrated, electrode 4 on spine A is in contact with cardiac tissues collected an electrocardiogram signal (indicated as "SPU A4" in Figure 7L) which typically includes signals from other areas of the heart (commonly referred to as "far field signals"). Because the counterpart electrode 3 on spine A is on the opposite side, and therefore not in tissue contact, the electrocardiogram of the counterpart electrode 3 (indicated as SPU A3, which is in the blood pool), includes collected far-field signals that can be used for subtraction (via suitable differential op-amps) from the collected signal A4. The resulting differential electrocardiogram signal (indicated here as "SPU A4-A3") provides for a high fidelity ECG signal with substantially all extraneous noise or far-field signals subtracted out to give an accurate ECG signal.

In this invention, however, applicant has taken this technique even further by combining separate closely spaced electrodes so as to form double reference electrodes for its counterpart pair of electrodes on the opposite surface. As viewed in the Table, A, B, C and D represent the respective spines with closely spaced electrode pair 1:3 on one surface 701a which has its counterpart pair 2:4 on the opposite side 701b. The same arrangement can be seen for the second electrode pair 5:7 and its counterpart electrode pair 6:8 and third electrode pair 9:11 with its counterpart electrode pair 10:12. With this arrangement, one closely spaced electrode pair on one surface can act as reference electrode pair (that receive far field signals by being in the blood pool without contacting tissue) to the other closely spaced pair of electrodes on the opposite facing surface that are in contact with tissues and vice versa.

In one example, assume that the top substrate or surface 701a is in contact with tissues with at least electrodes 1 and 3 in physical contact with tissues while bottom substrate or surface 701b is facing away from tissues so that at least electrodes 2 and 4 are not in physical contact with tissues. The subtraction of far field signals for this example on spine A can be as follows: signals from electrode A2 (not in tissue contact) are subtracted from electrode A1 (tissue contact), i.e., A1-A2. On the other hand, signals from electrode A4 (not in physical tissue contact) are subtracted from electrode A3 (in tissue contact). The noise subtraction can be represented symbolically as (A1-A2)-(A3-A4). The order of operation with the brackets for (A1-A2)-(A3-A4) can be reduced as A1-A3-A2+A4 to arrive at the same values. Where the order of tissue contact is reversed for the substrates in this example (with bottom substrate 701b in tissue contact and top substrate 701a in the blood pool), the same technique can be applied, i.e., (A2-A1)-(A4-A2) to achieve the high fidelity signal to noise from the electrodes. While this example uses spine A, it is noted that the same technique can be applied to the other pairs of closely-spaced electrodes on each of the remaining spines B, C, D and so on consistent with this disclosure.

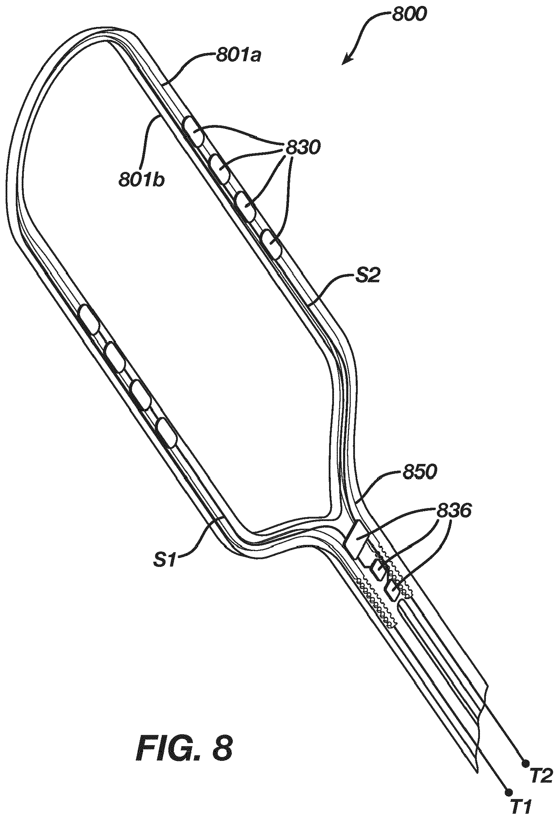

Figure 8 is an illustration of another example end effector 800 having a first side 801a, a second side 801b, and electrodes 830 on both sides 801a, 801b. Electrodes 830 on one side (of 801a or 801b) may act as a reference electrode(s) for counterpart electrodes 830 on the other side (of 801a or 801b). That is, when electrodes on one side (e.g., 801b) are in contact with tissues for sensing near-field signals (i.e., ECG), the electrodes on the opposite side (e.g., 801a) that are not in contact with the tissue can be used to sense far-field signals or noise such that the far-field noise can be subtracted from the near-field signals, thereby giving a very high fidelity ECG signals measured by the electrodes in contact with tissues. The end effector includes a frame 850. The frame 850 includes segments S1 and S2 connected to each other to form a conductive loop. Such conductive loop formed by frame 850 (or multiple frames such as in Figure 7F or 7J) may be connected to electrical terminals T1 and T2 in order for the frame to act as a magnetic location sensing coil as shown and described in US Patent Publication No. US2020/0289059A1, which is incorporated by reference herein. The end effector may include proximal reference electrodes 836.

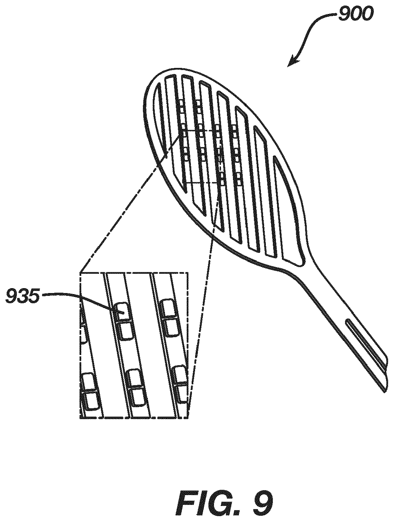

Figure 9 is an illustration of another example end effector 900 including closely spaced bipolar electrode pairs 935 which are similar in arrangements and dimensions to those described and illustrated in Figures 7F through 7L.

It is noted that the electrodes described and illustrated herein in relation to Figures 1A through 9 are not limited to mapping (i.e., sensing signals or recording signals) but can be used to deliver energy such as RF (alternating cycle) or IRE (DC pulses) in bipolar or unipolar mode alone or in combination with the mapping or sensing function. In the application for IRE, the electrodes can be configured to deliver at least 900V per electrode with a current of at least 20 amperes over a number of pulses sufficient to cause cell apoptosis.

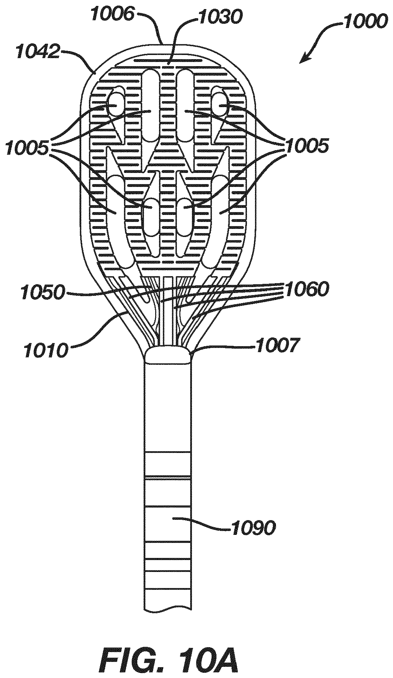

Figure 10A is an illustration of another example end effector 1000 having a large area electrode 1030. The large area electrode 1030 can be configured to deliver PFA/IRE ablation electrical signals to tissue, for instance as unipolar pulses between the large area electrode 1030 and one or more body patches applied to the skin of the patient PA (Figure 6). The large area electrode 1030 may be effective to ablate tissue to a greater tissue depth than smaller electrodes configured for PFA/IRE. The large area electrode 1030 may be effective to ablate thicker regions of the heart, such as in or near ventricles. The ablation surface of the large area electrode can measure between 70 and 110 mm², and more preferably, approximately 90 mm².

The large area electrode 1030 can be manufactured as a conductive layer of a flex circuit, laser cut from a sheet of electrically conductive material, direct printing of conductive ink, or other suitable means as understood by a person skilled in the pertinent art. As illustrated, the large area electrode can include interference cuts, or slits, to promote bending of the end effector 1000 into, and out of, the plane of the page. For instance, the large area electrode 1030 can be laser cut from a stainless steel foil. laser cut to include slits, and plated with platinum, gold, or other bio-compatible electrically conductive material.

The end effector 1000 can include one or more membranes 1010 and a polymer encapsulation 1042 similar the membranes 110, 120, 210, 220 and polymeric encapsulation 142 illustrated in Figures 1A though 2D, membranes and polymer encapsulation disclosed elsewhere herein, or variations thereof and alternatives thereto as understood by a person skilled in the pertinent art. The membrane(s) can include electrical conductors 1060 which extend into the shaft 1090 to provide electrical communication between the large area electrode 1030 and the console 612 (Figure 6). The end effector 1000 further includes a frame 1050 that is surrounded by the polymeric encapsulation 1042. The membrane(s) 1010 are affixed to the polymeric encapsulation 1042. The end effector 1000 can include two membranes, one on either side, similar to as illustrated in Figures 1A through 2D.

The membrane(s) 1010 can include a membrane on the illustrated side, and the membrane can define a generally planar surface extending distally from the distal end of a shaft 1090 of a catheter, from a proximal portion 1007 to a distal end 1006 of the end effector 1000. The large area electrode 1030 can occupy a majority of the generally planar surface. The end effector 1000 can include openings 1005 extending through the membrane(s) and polymeric encapsulation 1042 so that the openings 1005 extend entirely through the thickness of the end effector 1000.

The frame 1050 can include one or more struts configured similar to as illustrated in Figures 4A through 4C, as elsewhere disclosed herein, variations thereof, or alternatives thereto as understood by a person skilled in the pertinent art. The membrane(s) 1010 can extend between the one or more struts, and the large area electrode 1030 can be disposed on the illustrated membrane 1010 such that a majority of the large area electrode 1030 is positioned between the one or more struts of the frame 1050. For instance, the frame 1050 can include two struts as illustrated in Figure 1A or 4A, and the large area electrode 1030 can have a surface area portion that extends over the struts of the frame 1050 while a majority of the surface area of the large area electrode 1030 is disposed between the two struts of the frame 1050. In this way, at least a portion of a perimeter of the large area electrode 1030 is directly supported by the frame 1050, while the central portion of the large area electrode 1030 is not directly supported by the frame 1050.

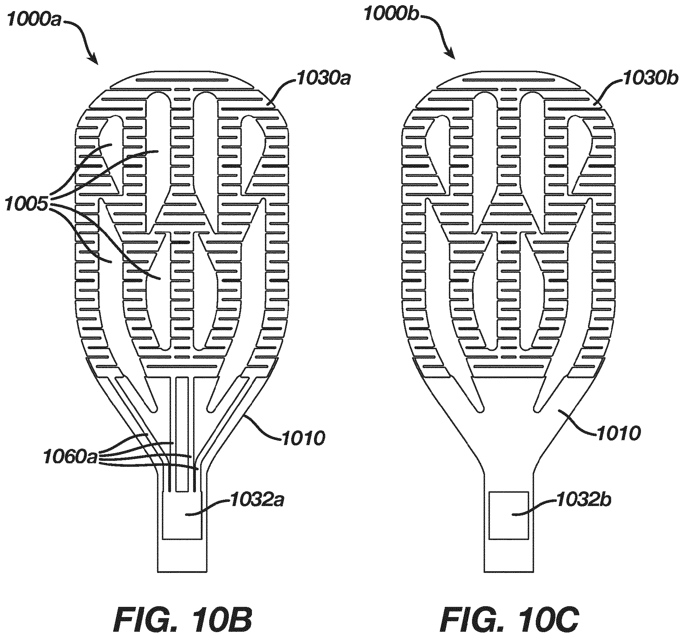

Figure 10B is an illustration of another example end effector 1000a having an example large area electrode 1030a. The large area electrode 1030a can be laser cut from a sheet and including interference cuts to promote bending of the end effector 1000 into and out of the plane of the page. The large area electrode 1030a can include a primary portion which occupies a majority of the surface area of the illustrated membrane 1010. The large area electrode 1030a can further include a proximal electrode portion 1032a that is disposed in a proximal direction in relation to the primary portion of the large area electrode 1030a. The large area electrode 1030a can include conductive traces or segments 1060a electrically and mechanically coupling the proximal portion 1032a of the large area electrode 1030a to the primary portion of the large area electrode 1030a. The example end effector 1000 can otherwise include features of end effector 1000 as described in relation to Figure 10A.

Figure 10C is an illustration of another example end effector 1000b having another example large area electrode 1030b and independent proximal electrode 1032b. The large area electrode 1030b can be configured as discussed in relation to the large area electrode 1030 illustrated in Figure 10A. The independent proximal electrode 1032b is electrically isolated from the large area electrode 1030b and can be configured to sense electrical signals from tissue and/or ablate independent of the large area electrode 1030b. Additionally, or alternatively, the end effector 1000b can be configured to provide bipolar ablation PFA/IRE signals between the independent proximal electrode 1032b and the large area electrode 1030b.

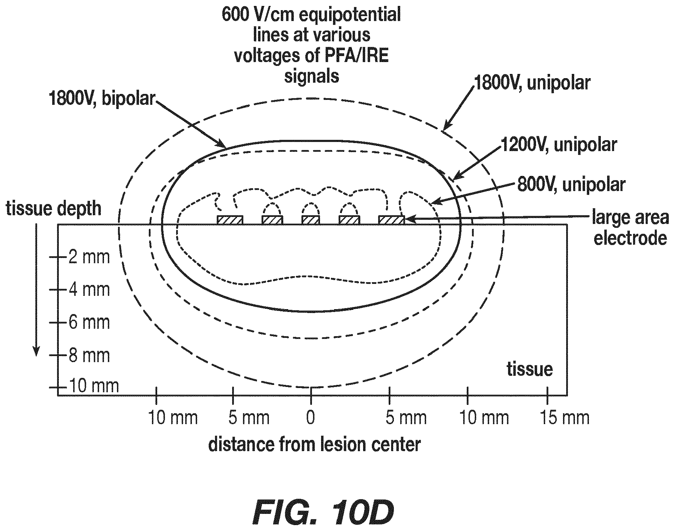

Figure 10D is an illustration of an example simulation of pulse field ablation signals applied to tissue by an example large area electrode as illustrated in Figures 10A through 10C. The illustration includes a box representative of tissue and five electrode portions disposed on a top surface of the tissue representative the large area electrode 1030. The illustration shows a cross section of tissue and the large area electrode 1030, for instance, as made across line X-X indicated in Figure 10C when the electrode 1030 is applied to tissue. The illustration includes 600 V/cm equipotential lines such that the magnitude of the electric field at each of the lines is 600 V/cm.

Electroporation can be induced by applying a pulsed electric field across biological cells to cause reversable (temporary) or irreversible (permanent) creation of pores in the cell membrane. Cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, IRE ablated cells die due to a loss of homeostasis and typically die by apoptosis . Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential. Figure 10D illustrates 600 V/cm equipotential lines, which is an approximate indication of expected lesion area of the various IRE ablation signals applied to heart cells.

To ablate using PFA/IRE, short duration voltage pulses are applied to disrupt cellular structures of myocardium. The pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area, which can lead to unintended damage of non-targeted tissue such as the esophagus. IRE therefore has the capability to spare adjacent heat sensitive structures or tissues which would be of benefit in the reduction of possible complications known with ablation or isolation modalities.

The different ablation modalities of RF ablation vs IRE ablation generally results smaller and shallower lesion creation with IRE vs. RF for a similar ablation electrode configuration. Because RF ablation generates thermal damage, lesion size continues to increase due to thermal conduction of heath through tissue after the application of the RF ablation signal is removed from the tissue. IRE ablation is non-thermal, and therefore lesion size does not significantly increase after the ablation signal is removed from the tissue and may contract in size due to self-healing of cells in the lesion outer region which experience reversible electroporation. Prior to this disclosure, IRE catheters have been demonstrated to be capable of creating only shallow lesions, which may be adequate for ablating atrium tissue, but are not adequate for ablating thicker heart tissue, such as at or near ventricles.

As a solution to this problem, the large area electrode 1030 provides a significantly larger surface area which is configured to contact tissue during ablation. In a small area electrode, typical of previously demonstrated IRE catheters, the electric field intensity is approximately inversely proportionate to the square of the distance from the small area electrode, meaning that voltage applied to the small area electrode must be exponentially increased to achieve a linear increase in lesion depth. The large area electrode 1030 has a surface area such that in tissue regions immediately adjacent the large area electrode 1030, electric field lines extend substantially parallel to each other, orthogonal to the plane of the large area electrode 1030 over a significant portion of the central region of the large area electrode 1030.

Figure 10D illustrates a large area electrode 1030 having a total width of approximately 12 mm and divided into five segments having spaces therebetween. As illustrated, the large area electrode 1030 is capable of creating a lesion having a size indicated by the equipotential lines, based on the pulse voltage (1,800 V, 1,200 V, or 800 V) and electrode configuration (unipolar, bipolar). For an IRE signal having 1,800 V unipolar pulses, the lesion depth is approximately 10 mm and the lesion width is approximately 25 mm. For an IRE signal having 1,200 V unipolar pulses, the lesion depth is approximately 7 mm and the lesion width is approximately 21 mm. For an IRE signal having 800 V unipolar pulses, the lesion depth is approximately 4 mm and the lesion width is approximately 12 mm. For an IRE signal having 1,800 V bipolar pulses, the lesion depth is approximately 5 mm and the lesion width is approximately 18 mm.

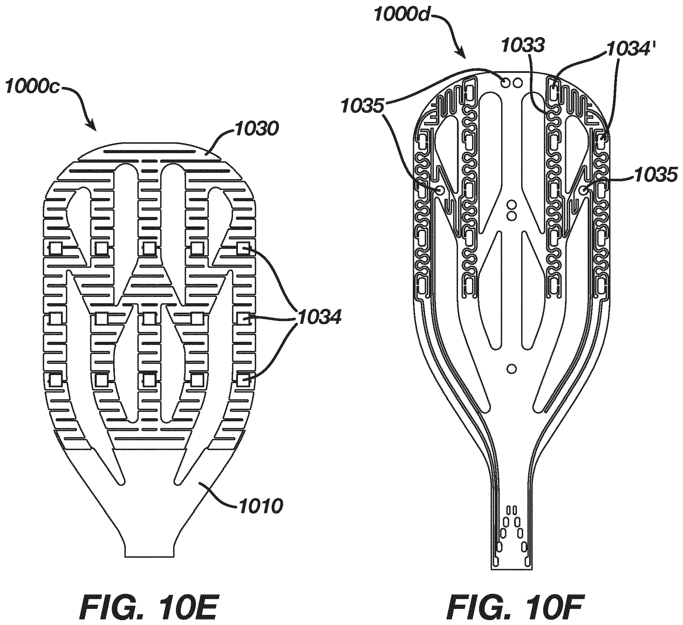

Figure 10E is an illustration of another example end effector 1000c, which is a variation of the example end effector 1000 of Figures 10A through 10C including smaller, independent electrodes 1034. The independent electrodes 1034 can be electrically isolated from the large area electrode 1030, and can be configured to sense electrical signals from tissue. During an ablation procedure, the independent electrodes 1034 can be used to detect the effectiveness of lesion creation at interrupting an unwanted electrical pathway through cardiac tissue. The end effector 1000c can maintain its position at a target site while delivering a first ablation signal by the large area electrode 1030, monitor for electrical signals through tissue by the independent electrodes 1034, and apply a second ablation signal by the large area electrode 1030 if unwanted electrical signals are detected by the independent electrodes 1034.

The independent electrodes 1034 can be spaced with various arrangements. For instance, the independent electrodes 1034 can be spaced individually as illustrated in Figure 10E. Additionally, or alternatively, at least a portion of the independent electrodes 1034 can be spaced in a bipolar pair arrangement such as electrode pair 235 illustrated in Figure 2B, electrode pairs 730a1, 730b1 illustrated in Figure 7F, electrode pairs 5:7, 1:3 illustrated in Figure 7K, or variation thereof as understood by a person skilled in the pertinent art. The end effector 1000c can include electrodes on a back, or opposite side of the end effector (not shown in the illustration) that are configured to contact blood or other body fluids while the independent electrodes 1034 are in contact with tissue. The back side electrode(s) can function as a reference electrode to the independent electrodes 1034. The end effector 1000c can include a plurality of backside electrodes that are directly opposite at least a portion of the independent electrodes 1034. The independent electrodes 1034 and backside electrodes can be configured such that far field noise can be filtered from an ECG signal detected by the independent electrodes 1034 similar to as described in relation to Figure 7L, or through other methods as understood by a person skilled in the pertinent art.

The end effector 1000 illustrated in Figure 10A, and variations 1000a, 1000b, 1000c, 1000d thereof illustrated in Figures 10B, 10C, and 10E can include a second large area electrode 1030, or variation thereof, on a back, or opposite side of the end effector (not shown in illustrations). Additionally, or alternatively, the back side of the end effector 1000c can include electrodes arranged or otherwise configured similar to electrodes 130a illustrated in Figure 1A, electrode pairs 235 illustrated in Figure 2B, or variation thereof as understood by a person skilled in the pertinent art.

Figure 10F illustrates a variation of end effector 1000d in which mapping or recording electrodes 1034' are provided alongside tissue contact quality electrodes 1035 can be provided to ensure that the signals recorded by mapping electrodes 1034' are in contact with tissues as verified by impedance measured by tissue contact electrodes 1035.

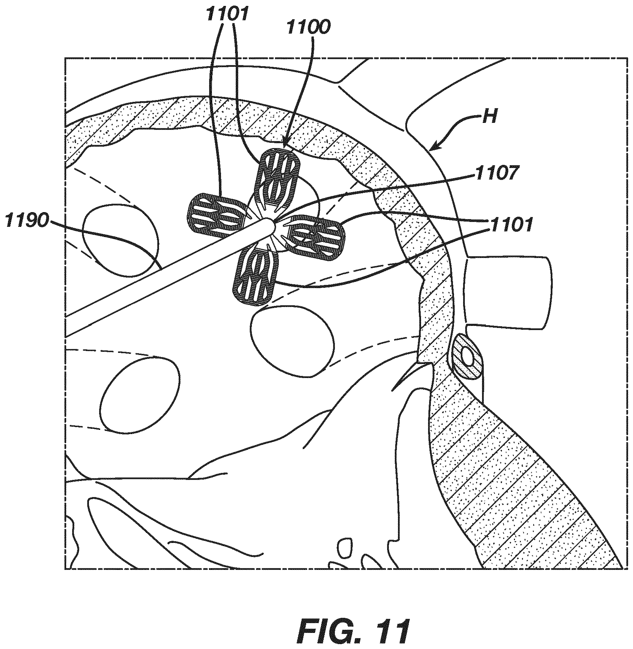

Figure 11 is an illustration of another example end effector 1100 including multiple membrane structures 1101. Each membrane structure 1101 can be formed, shaped, and otherwise configured similar to the end effector 100 illustrated in Figure 1A, the end effector 200 illustrated in Figure 2A, the end effector illustrated in Figure 3, the end effector 1000 illustrated in Figure 10A, and/or variations thereof. Each membrane structure 1101 can include a frame, at least one membrane extending between struts of the frame, and at least one electrode disposed on a membrane and positioned between the struts. Each membrane structure can further include a polymeric encapsulation surrounding the frame. Each membrane structure can include additional features including one or more piezoelectric sensors, one or more navigation sensors, and other compatible features of aforementioned end effectors 100, 200, 1000. The membrane structures can sense and/or ablate by utilizing electrodes configured similar to as described in relation to end effectors 100, 200, 1000.

Any of the examples or embodiments described herein may include various other features in addition to or in lieu of those described above. The teachings, expressions, embodiments, examples, etc. described herein should not be viewed in isolation relative to each other. Various suitable ways in which the teachings herein may be combined should be clear to those skilled in the art in view of the teachings herein.

The following clauses list non-limiting embodiments of the disclosure:
Clause 1. An end effector of a catheter, the end effector comprising: a frame comprising one or more struts extending along a longitudinal axis; a first membrane extending between the one or more struts on a first side of the end effector to define a generally planar first surface exposed to ambient environment; a plurality of first electrodes affixed to the first membrane at predetermined locations on the first surface; a second membrane extending between the one or more struts such that at least a portion of the frame is between the first membrane and the second membrane, the second membrane defining a generally planar second surface exposed to the ambient environment; and a plurality of second electrodes affixed to the second surface so that each second electrode is disposed substantially opposite each first electrode and spaced apart from each other.
Clause 2. The end effector of clause 1, the frame being configured symmetrical about the longitudinal axis.
Clause 3. The end effector of clause 1 or 2, the one or more struts comprising a first strut and a second strut each positioned between the first membrane and the second membrane.
Clause 4. The end effector of clause 3, the first and second struts being symmetric about the longitudinal axis.
Clause 5. The end effector of any one of clauses 1-4, the one or more struts comprising a proximal strut and a pair of struts extending distally from the proximal strut.
Clause 6. The end effector of clause 5, the pair of struts each terminating at a respective distal strut end.
Clause 7. The end effector of clause 5 or 6, the proximal strut comprising a width greater than a width of each strut of the pair of struts.
Clause 8. The end effector of any one of clauses 1-7, the one or more struts comprising a proximal strut and between two and twelve struts extending distally from the proximal strut.
Clause 9. The end effector of any one of clauses 1-8, the one or more struts comprising one or more looped struts positioned approximate a perimeter of the end effector, the one or more looped struts comprising a connected looped strut and/or a disconnected looped strut.
Clause 10. The end effector of clause 9 further comprising: electrical conductors in electrical contact with the looped strut and extending proximally from the frame.
Clause 11. The end effector of clause 9 or 10, the looped strut being symmetric about the longitudinal axis.
Clause 12. The end effector of any one of clauses 9-11, the looped strut comprising a first proximal end, a second proximal end proximal, and a continuous, non-branching path extending from the first proximal end to the second proximal end.
Clause 13. The end effector of any one of clauses 9-12, the looped strut comprising a pair of parallel segments extending parallel to the longitudinal axis and a connector segment extending across the longitudinal axis and joining segments of the pair of parallel segments.
Clause 14. The end effector of any one of clauses 1-13, at least a portion of the one or more struts comprising a flexibility gradient along a portion of a length of the respective strut.
Clause 15. The end effector of any one of clauses 1-14, at least a portion of the one or more struts comprising a graduated cutout pattern along a portion of a length of the respective strut.
Clause 16. The end effector of any one of clauses 1-15, at least one of the first membrane and the second membrane comprising a flex circuit.
Clause 17. The end effector of any one of clauses 1-16, the first membrane comprising electrically conductive traces in electrical contact with the plurality of first electrodes, and the second membrane comprising electrically conductive traces in electrical contact with the plurality of second electrodes.
Clause 18. The end effector of any one of clauses 1-17, the first membrane and the second membrane each comprising a respective proximal extension extending proximally from a proximal end of the frame.
Clause 19. The end effector of clause 18, the proximal extensions comprising a length of between about 30 centimeters (cm) and about 150 cm.
Clause 20. The end effector of clause 18 or 19, the proximal extensions comprising a plurality of conductive traces in electrical contact with the plurality of first electrodes and the plurality of second electrodes.
Clause 21. The end effector of any one of clauses 1-20, at least a portion of the plurality of first electrodes being positioned to contact tissue.
Clause 22. The end effector of any one of clauses 1-21, at least a portion of the plurality of first electrodes being positioned between the one or more struts.
Clause 23. The end effector of any one of clauses 1-22, the plurality of first electrodes comprising a gap between a pair of coplanar electrodes measuring about 5 micrometers to about 650 micrometers.
Clause 24. The end effector of any one of clauses 1-23, the plurality of first electrodes comprising a plurality of closely spaced coplanar electrode pairs.
Clause 25. The end effector of clause 24, the closely spaced coplanar electrode pairs being spaced with a pitch of between about 0.5 millimeters and about 6 millimeters between pairs and a gap of about 150 micrometers between electrodes of a pair.
Clause 26. The end effector of any one of clauses 1-25, the first membrane comprising a first longitudinally extending section comprising a first width, a first electrode of the plurality of first electrodes affixed to the first membrane being disposed centrally in relation to the first width, the first membrane comprising a second longitudinally extending section comprising a second width greater than the first width, and a second electrode of the plurality of first electrodes affixed to the first membrane being disposed centrally in relation to the second width.
Clause 27. The end effector of any one of clauses 1-26, in which one of the pluralities of the first electrodes and second electrodes that are not in contact with tissue comprising a reference electrode.
Clause 28. The end effector of any one of clauses 1-26, in which a plurality of the first and second electrodes that are not in contact with tissue include a plurality of reference electrodes each configured to provide a reference signal to a plurality of the other of the first and second electrodes that are in contact with tissue.
Clause 29. The end effector of any one of clauses 1-28, the plurality of first electrodes being distributed in a high electrode density zone and low electrode density zone, the high electrode density zone being disposed distal of the low electrode density zone, and the high electrode density zone being of approximately equal area as the low electrode density zone.
Clause 30. The end effector of any one of clauses 1-29, further comprising: a reference electrode positioned on a portion of the end effector configured to be positioned approximate a distal end of a catheter shaft.
Clause 31. The end effector of any one of clauses 1-30, further comprising: a polymer encapsulating the frame.
Clause 32. The end effector of any one of clauses 1-31, further comprising: a polymer being positioned between the first membrane and the second membrane.
Clause 33. The end effector of any one of clauses 1-32, further comprising: a navigation sensor positioned such that at least a portion of the navigation sensor is between the one or more struts.
Clause 34. The end effector of clause 33, the navigation sensor comprising an area of about 16 millimeters squared and being configured as a single axis sensor or a double axis sensor.
Clause 35. The end effector of any one of clauses 1-34, further comprising: one or more piezoelectric transducers.
Clause 36. The end effector of clause 35, the one or more piezoelectric transducers being disposed under a respective electrode of the plurality of first electrodes.
Clause 37. The end effector of clause 35 or 36, the one or more piezoelectric transducers being positioned within an opening in a strut of the one or more struts.
Clause 38. The end effector of any one of clauses 35-37, the one or more piezoelectric transducers being positioned between the first membrane, between the second membrane, and between the one or more struts.
Clause 39. The end effector of any one of clauses 35-38, the one or more piezoelectric transducers comprising lead zirconate titanate (PZT).
Clause 40. The end effector of any one of clauses 35-39, the one or more piezoelectric transducers being configured as an ultrasound transducer.
Clause 41. The end effector of any one of clauses 1-40, in which each of the first membrane and second membrane comprises a continuous single member.
Clause 42. The end effector of any one of clauses 1-41, in which each of the first membrane and second membrane includes a cut-out disposed on at least one side of the longitudinal axis.
Clause 43. The end effector of any one of clauses 1-42, further comprising: a pair of longitudinal openings separating a central portion of the end effector from a left portion of the end effector and a right portion of the end effector.
Clause 44. The end effector of clause 43, the frame being positioned in the left portion and the right portion, and the frame being absent in the central portion.
Clause 45. The end effector of clause 43 or 44, the end effector being configured for delivery through a catheter comprising an inner diameter, and the central portion comprising a width approximately equal to the inner diameter of the catheter.
Clause 46. The end effector of any one of clauses 43-45, the left portion and the right portion being configured to fold toward the central portion for delivery of the end effector through a catheter.
Clause 47. The end effector of any one of clauses 43-46, a majority of the plurality of first electrodes being disposed on the central portion of the end effector, a first portion of the plurality of first electrodes being disposed on the right portion of the end effector, and va second portion of the plurality of first electrodes being disposed on the left portion of the end effector.
Clause 48. The end effector of any one of clauses 43-47, the central portion comprising a plurality of openings each being longitudinally shorter in length than each of the pair of longitudinal openings.
Clause 49. The end effector of any one of clauses 43-48, the frame comprising a first distal end positioned in the left portion, the frame comprising a second distal end positioned in the right portion, at least one of the first membrane and the second membrane extending over the first distal end of the frame, across a distal end of the end effector, and over the second distal end of the frame.
Clause 50. The end effector of clause 49, at least one of the first membrane and the second membrane extending in a single arc from the first distal end of the frame, across the distal end of the end effector, and to the second distal end of the frame.
Clause 51. The end effector of clause 49 or 50, at least one of the first membrane and the second membrane extending in a first arc from the first distal end of the frame to the central portion of the end effector, a second arc across a distal end of the central portion, and a third arc from the central portion to the second distal end of the frame.
Clause 52. The end effector of any one of clauses 1-51, the end effector, in free space, comprising a curvature defining an arcuate path around the longitudinal axis.
Clause 53. The end effector of clause 52, the end effector, pressed to a planar surface, comprising a planar shape.
Clause 54. A catheter control system comprising: a processor; and non-transitory computer readable medium in communication with the processor and comprising instructions thereon that when executed by the processor causes the system to: receive a mapping electrical signal from a mapping electrode such that the mapping electrode faces tissue in a first direction and is disposed on a first side of an end effector of a catheter, receive a reference electrical signal from a reference electrode such that the reference electrode faces away from the tissue in a second direction and is disposed on a second side of the end effector opposite the first side, and receive an ultrasound electrical signal from an acoustic transducer disposed in a plane that is between the mapping electrode and the reference electrode.
Clause 55. A catheter control system comprising: a processor; and non-transitory computer readable medium in communication with the processor and comprising instructions thereon that when executed by the processor causes the system to: receive a mapping electrical signal from a mapping electrode such that the mapping electrode faces tissue in a first direction and is disposed on a first side of an end effector of a catheter, receive a reference electrical signal from a reference electrode such that the reference electrode faces away from the tissue in a second direction and is disposed on a second side of the end effector opposite the first side, apply an alternating current to a first plurality of tomography electrodes disposed on the first side of the end effector, receive equi-potentials of a second plurality of tomography electrodes paired with the first plurality of tomography electrodes and disposed on the first side of the end effector, generate a tomographic image of the tissue based on the equi-potentials, and display the tomographic image on a display.
Clause 56. The catheter control system of clause 54 or 55, the non-transitory computer readable medium further comprising instructions thereon that when executed by the processor causes the system to: receive a bipolar mapping electrical signal from a first pair of bipolar electrodes facing the first direction on the first surface, and receive the reference electrical signal from a second pair of reference electrodes disposed opposite the first pair of bipolar electrodes such the second pair of reference electrodes face the second direction opposite the first direction on the second side of the end effector.
Clause 57. The catheter control system of any one of clauses 54 through 56, the non-transitory computer readable medium further comprising instructions thereon that when executed by the processor causes the system to: receive a navigation signal from a single axis sensor coil disposed in a plane that is between the mapping electrode and the reference electrode.
Clause 58. The catheter control system of any one of clauses 54 through 57, the non-transitory computer readable medium further comprising instructions thereon that when executed by the processor causes the system to: receive a navigation signal from a single axis sensor coil disposed at a distal end of a shaft of the catheter and disposed in an off-angle plane that is at an angle to an end effector plane that is between the mapping electrode and the reference electrode.
Clause 59. The catheter control system of any one of clauses 54 through 58, the non-transitory computer readable medium further comprising instructions thereon that when executed by the processor causes the system to: determine a positioned of the end effector based at least in part on an impedance between an electrode of the end effector and a body patch electrode.
Clause 60. A method of constructing a catheter, the method comprising: inserting a distal end of an assembled end effector into a proximal end of a catheter shaft; moving the distal end of the assembled end effector through an entire length of the catheter shaft; moving the distal end of the assembled end effector out of a distal end of the catheter shaft; and securing the assembled end effector to the catheter shaft.
Clause 61. The method of clause 60, wherein the assembled end effector comprises an electronic circuit at a proximal end of the assembled end effector.
Clause 62. The method of clause 61, further comprising: affixing a catheter handle around the electronic circuit.
Clause 63. An end effector configured for a catheter, the end effector comprising: a frame structure (750a) comprising first and second segments (S1, S2) extending alongside each other in a direction along a longitudinal axis; each segment having a first outer surface and a second outer surface facing away in a generally opposite direction to the first outer surface; the first outer surface includes at least one first pair of closely-spaced electrodes in which each first pair of closely-spaced electrodes includes two electrodes spaced apart over any gap length of approximately 0.1 to approximately 0.4 mm as measured from one electrode edge of one electrode to a nearest electrode edge of the other closely-spaced electrode; and the second outer surface includes at least a second pair of closely-spaced electrodes in which each second pair of closely-spaced electrodes includes two electrodes spaced apart over any gap length of approximately 0.1 mm to approximately 0.4 mm from one electrode edge of one electrode to a nearest electrode edge of the other closely-spaced electrode.
Clause 64. The end effector of clause 63, in which the first outer surface comprises a surface substantially parallel to the second outer surface.
Clause 65. The end effector of clause 63, in which the two segments are connected to each other at one end to form a conductive loop, the conductive loop having electrical terminations at a proximal portion so that the conductive loop comprises an electromagnetic sensor.
Clause 66. The end effector of clause 63, in which the at least one first pair of closely-spaced electrodes on the first surface comprise two pairs of closely spaced electrodes in which one pair of closely-spaced electrodes is separated to the other pair of closely-spaced electrodes by a gap length (Ls) measured from the closest edges of respective electrodes of any value from approximately 0.5mm to 1.5 mm.
Clause 67. The end effector of clause 63, in which the at least one second pair of closely-spaced electrodes on the second surface comprise two pairs of closely spaced electrodes in which one pair of closely-spaced electrodes is separated to the other pair of closely-spaced electrodes by a gap length (Ls) measured from the closest edges of respective electrodes of any value from approximately 0.5mm to 2 mm.
Clause 68. The end effector of any clauses of clauses 63-67 in which each electrode comprises a surface area of any value from approximately 0.04 mm-squared to 1 mm-squared in surface area exposed to ambient environment.
Clause 69. The end effector of any clauses of clauses 63-68 in which each electrode comprises a rectangular area exposed to ambient environment having a length along the longitudinal axis of any value from approximately 0.1 mm to approximately 1 mm and a width of any value from approximately 0.1 mm to approximately 1 mm.
Clause 70. The end effector of any clauses of clauses 63-68 in which each electrode comprises a square area exposed to ambient environment having a side of any value from approximately 0.1 mm to approximately 1 mm.
Clause 71. The end effector of any clauses of clauses 63-68 in which each electrode comprises a non-linear surface area.
Clause 72. The end effector of any clauses of clauses 63-70 in which each electrode on the first outer surface includes an electrode surface exposed to the ambient environment parallel to a counterpart electrode surface of a counterpart electrode disposed on the second outer surface.
Clause 73. The end effector of any clauses 63-70 in which each segment comprises a linear cross-sectional area orthogonal to the longitudinal axis.
Clause 74. The end effector of any clauses 63-70 in which each segment comprises a non-linear cross-sectional area orthogonal to the longitudinal axis.
Clause 75. The end effector of any clauses 63-70 in which each electrode comprises a linear cross-sectional area orthogonal to the longitudinal axis, the area from about 0.00005mm-squared to about 0.005 mm-squared.
Clause 76. The end effector of any clauses 63-70 in which each electrode comprises a non-linear cross-sectional area orthogonal to the longitudinal axis.
Clause 77. The end effector of any clauses 63-70 further comprising another frame structure (750b) having third and fourth segments (S3, S4) extending along side each other and the first and second segments so that the third segment (S3) is disposed between the first and second segments (S1, S2) and the second segment (S2) is disposed between the third and fourth segments (S3, S4).
Clause 78. The end effector of clause 77 in which an electrode on one segment is spaced apart from a center of the electrode to a center of another electrode on adjacent segment over a first transverse gap (Lt1) having any value from approximately 0.5 mm to approximately 4 mm.
Clause 79. The end effector of clause 78, in which an electrode on one segment is spaced apart from the electrode edge to a nearest electrode edge of another electrode on adjacent segment over a second transverse gap (Lt2) having any value from approximately 0.3mm to approximately 3.8 mm.
Clause 80. The end effector of clause 79, in which one segment is spaced apart from another segment along the transverse axis over a third transverse gap (Lt3) of less than the second transverse gap (Lt2).
Clause 81. The end effector of clause 80, in which all the segments are parallel to each other.
Clause 82. An end effector of a catheter, the end effector comprising: a frame comprising one or more struts extending along a longitudinal axis; a first surface extending along each of the one or more struts on a first side of the end effector to define a generally planar first substrate exposed to ambient environment; a pair of closely-spaced first electrodes affixed to the first surface, the closely-spaced first pair of electrodes comprises a first electrode and a second electrode spaced apart from edge to edge of less than 1 mm; and a pair of closely-spaced second electrodes affixed to the second surface opposite the first surface, the closely-spaced second pair of electrodes comprises a third electrode disposed with its center in substantial alignment with the center of the first electrode on the first surface and a fourth electrode with its center disposed in substantially alignment with the center of the second electrode so that when one of the first and second pairs of electrodes are in contact with tissue to receive tissue signals and the other of the first and second pairs of electrodes are not in contact with tissue to receive far-field signals configured for subtraction of far-field signals contained in the tissue signals by the pair of electrodes in contact with tissues.
Clause 83. An end effector of a catheter, the end effector comprising: a frame comprising one or more struts extending along a longitudinal axis; a polymeric encapsulation surrounding a majority of the frame of the end effector; a first membrane extending between the one or more struts on a first side of the end effector to define a generally planar first surface exposed to ambient environment; and at least one first electrode disposed on the first membrane such that a majority of the at least one first electrode is positioned between the one or more struts of the frame.
Clause 84. The end effector of clause 83, wherein the at least one first electrode comprises a plurality of first electrodes, the end effector further comprising: a second membrane extending between the one or more struts such that at least a portion of the frame is between the first membrane and the second membrane, the second membrane defining a generally planar second surface exposed to the ambient environment; and a plurality of second electrodes affixed to the second surface so that each second electrode is disposed substantially opposite each first electrode and spaced apart from each other at predetermined locations.
Clause 85. The end effector of clause 84, in which one of the pluralities of the first electrodes and second electrodes that are not in contact with tissue comprising a reference electrode.
Clause 86. The end effector of clause 84 or 86, wherein the polymeric encapsulation is positioned between the first membrane and the second membrane.
Clause 87. The end effector of any one of clauses 84-86, the one or more struts comprising a proximal strut and a pair of struts extending distally from the proximal strut.
Clause 88. The end effector of any one of clauses 84-87, the one or more struts comprising a proximal strut and between two and twelve struts extending distally from the proximal strut.
Clause 89. The end effector of any one of clauses 83-88, the one or more struts comprising one or more looped struts positioned approximate a perimeter of the end effector, the one or more looped struts comprising a connected loop strut and/or a disconnected loop strut, and the looped strut comprising a first proximal end, a second proximal end proximal, and a continuous, non-branching path extending from the first proximal end to the second proximal end.
Clause 90. The end effector of any one of clauses 83-89, at least a portion of the one or more struts comprising a flexibility gradient along a portion of a length of the respective strut.
Clause 91. The end effector of any one of clauses 83-90, the first membrane comprising a flex circuit.
Clause 92. The end effector of any one of clauses 83-91, the at least one first electrode being positioned to contact tissue.
Clause 93. The end effector of any one of clauses 83-92, the at least one first electrode comprising a plurality of closely spaced coplanar electrode pairs.
Clause 94. The end effector of any one of clauses 83-93, the first membrane comprising a first longitudinally extending section comprising a first width, the first membrane comprising a second longitudinally extending section comprising a second width greater than the first width, the at least one first electrodes comprising two electrodes such that one of the two electrodes is affixed to the first membrane and disposed centrally in relation to the first width and the other of the two electrodes is affixed to the first membrane and disposed centrally in relation to the second width.
Clause 95. The end effector of any one of clauses 83-94, the at least one first electrode comprising a plurality of first electrodes distributed in a high electrode density zone and low electrode density zone, the high electrode density zone being disposed distal of the low electrode density zone, and the high electrode density zone being of approximately equal area as the low electrode density zone.
Clause 96. The end effector of any one of clauses 83-95, further comprising: a navigation sensor positioned such that at least a portion of the navigation sensor is between the one or more struts.
Clause 97. The end effector of any one of clauses 83-96, further comprising: one or more piezoelectric transducers disposed under a respective electrode of the at least one of first electrode.
Clause 98. The end effector of any one of clauses 83-97, the end effector, in free space, comprising a curvature defining an arcuate path.
Clause 99. The end effector of clause 98, the end effector, pressed to a planar surface, comprising a planar shape.
Clause 100. The end effector of any one of clauses 83-99, wherein the at least one first electrode comprises a singular electrode having a surface area over a majority of the generally planar first surface.
Clause 101. The end effector of clause 100, wherein the at least one first electrode comprises a plurality of smaller electrodes disposed at predetermined locations on the second surface, the plurality of smaller electrodes being electrically isolated from the singular electrode.
Clause 102. The end effector of any one of clauses 83-101, further comprising: a first membrane structure comprising the frame, the polymeric encapsulation, the first membrane, and the at least one first electrode; and one or more additional membrane structures, each comprising a respective frame comprising respective one or more struts, a respective polymeric encapsulation surrounding a majority of the respective frame, a respective membrane extending between the one or more respective struts of the respective frame, and at least one respective electrode positioned between the one or more respective struts.

Having shown and described exemplary embodiments of the subject matter contained herein, further adaptations of the methods and systems described herein may be accomplished by appropriate modifications without departing from the scope of the claims. In addition, where methods and steps described above indicate certain events occurring in certain order, it is intended that certain steps do not have to be performed in the order described but, in any order, as long as the steps allow the embodiments to function for their intended purposes. Therefore, to the extent there are variations of the invention, which are within the spirit of the disclosure or equivalent to the inventions found in the claims, it is the intent that this patent will cover those variations as well. Some such modifications should be apparent to those skilled in the art. For instance, the examples, embodiments, geometrics, materials, dimensions, ratios, steps, and the like discussed above are illustrative. Accordingly, the claims should not be limited to the specific details of structure and operation set forth in the written description and drawings.

## Claims

1. An end effector of a catheter, the end effector comprising:
a frame comprising one or more struts extending along a longitudinal axis;
a polymeric encapsulation surrounding a majority of the frame;
a first membrane extending between the one or more struts on a first side of the end effector to define a generally planar first surface exposed to ambient environment; and
at least one first electrode disposed on the first membrane such that a majority of the at least one first electrode is positioned between the one or more struts of the frame.

2. The end effector of claim 1, wherein the at least one first electrode comprises a plurality of first electrodes, the end effector further comprising:
a second membrane extending between the one or more struts such that at least a portion of the frame is between the first membrane and the second membrane, the second membrane defining a generally planar second surface exposed to the ambient environment; and
a plurality of second electrodes affixed to the second surface so that each second electrode is disposed substantially opposite each first electrode and spaced apart from each other at predetermined locations.

3. The end effector of claim 2, in which one of the pluralities of the first electrodes and second electrodes that are not in contact with tissue comprising a reference electrode.

4. The end effector of claim 2 or claim 3, wherein the polymeric encapsulation is positioned between the first membrane and the second membrane.

5. The end effector of any of claims 1 to 4, the one or more struts comprising a proximal strut and a pair of struts extending distally from the proximal strut, or the one or more struts comprising a proximal strut and between two and twelve struts extending distally from the proximal strut.

6. The end effector of any preceding claim, the one or more struts comprising one or more looped struts positioned approximate a perimeter of the end effector, the one or more looped struts comprising a connected loop strut and/or a disconnected loop strut, and the looped strut comprising a first proximal end, a second proximal end proximal, and a continuous, non-branching path extending from the first proximal end to the second proximal end.

7. The end effector of any preceding claim, at least a portion of the one or more struts comprising a flexibility gradient along a portion of a length of the respective strut.

8. The end effector of any preceding claim, the first membrane comprising a flex circuit, the flex circuit including serpentine traces connected to the at least one first or second electrode.

9. The end effector of claim 1, the at least one first electrode being positioned to contact tissue, and/or comprising a plurality of closely spaced coplanar electrode pairs.

10. The end effector of any preceding claim,
the first membrane comprising a first longitudinally extending section comprising a first width,
the first membrane comprising a second longitudinally extending section comprising a second width greater than the first width,
the at least one first electrodes comprising two electrodes such that one of the two electrodes is affixed to the first membrane and disposed centrally in relation to the first width and the other of the two electrodes is affixed to the first membrane and disposed centrally in relation to the second width.

11. The end effector of any preceding claim,
the at least one first electrode comprising a plurality of first electrodes distributed in a high electrode density zone and low electrode density zone,
the high electrode density zone being disposed distal of the low electrode density zone, and
the high electrode density zone being of approximately equal area as the low electrode density zone.

12. The end effector of claim 1, further comprising:
a navigation sensor positioned such that at least a portion of the navigation sensor is between the one or more struts, and/or one or more piezoelectric transducers disposed under a respective electrode of the at least one of first electrode.

13. The end effector of claim 1, the end effector, in free space, comprising a curvature defining an arcuate path, optionally the end effector, pressed to a planar surface, comprising a planar shape.

14. The end effector of any preceding claim, wherein the at least one first electrode comprises a singular electrode having a surface area over a majority of the generally planar first surface., optionally wherein the at least one first electrode comprises a plurality of smaller electrodes disposed at predetermined locations on the generally planar first surface, the plurality of smaller electrodes being electrically isolated from the singular electrode.

15. The end effector of claim 1, further comprising:
a first membrane structure comprising the frame, the polymeric encapsulation, the first membrane, and the at least one first electrode; and
one or more additional membrane structures, each comprising a respective frame comprising respective one or more struts, a respective polymeric encapsulation surrounding a majority of the respective frame, a respective membrane extending between the one or more respective struts of the respective frame, and at least one respective electrode positioned between the one or more respective struts.
